(19) Europäisches Patentamt
European Patent Office
Office européen des brevets

(11) **EP 3 551 232 B1**

(12) **EUROPEAN PATENT SPECIFICATION**

(45) Date of publication and mention of the grant of the patent:
**23.03.2022 Bulletin 2022/12**

(21) Application number: **17838149.7**

(22) Date of filing: **07.12.2017**

(51) International Patent Classification (IPC):
***A61K 51/04*** *(2006.01)*

(52) Cooperative Patent Classification (CPC):
**A61K 51/0406; A61K 51/121**

(86) International application number:
**PCT/IB2017/057720**

(87) International publication number:
**WO 2018/104900 (14.06.2018 Gazette 2018/24)**

# (54) STABLE F-DOPA FORMULATIONS AND USES THEREOF

STABILE F-DOPA-FORMULIERUNGEN UND VERWENDUNGEN DAVON

FORMULATIONS STABLES DE F-DOPA ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2016 US 201662431187 P**

(43) Date of publication of application:
**16.10.2019 Bulletin 2019/42**

(73) Proprietors:
- **Università degli Studi di Bari "Aldo Moro" 70121 Bari (BA) (IT)**
- **ITEL Telecomunicazioni S.r.l. 70037 Ruvo di Puglia (BA) (IT)**

(72) Inventors:
- **SCILIMATI, Antonio 70125 Bari (IT)**
- **DENORA, Nunzio 70125 Bari (IT)**
- **TRICARICO, Domenico 70125 Bari (IT)**
- **DIAFERIA, Michele 70037 Ruvo di Puglia (BA) (IT)**

(74) Representative: **Freyria Fava, Cristina Buzzi, Notaro & Antonielli d'Oulx S.p.A. Corso Vittorio Emanuele II, 6 10123 Torino (IT)**

(56) References cited:
**WO-A1-2006/037950 CN-A- 107 198 780
US-A1- 2012 029 085 US-A1- 2015 157 743**

- **KANEKO S ET AL: "Enzymatic synthesis of no-carrier-added 6-[<18>F]fluoro-l-dopa with beta-tyrosinase", APPLIED RADIATION AND ISOTOPES, ELSEVIER, OXFORD, GB, vol. 50, no. 6, 1 June 1999 (1999-06-01), pages 1025-1032, XP004162250, ISSN: 0969-8043, DOI: 10.1016/S0969-8043(98)00173-0**
- **TRAPANI ADRIANA ET AL: "A novel injectable formulation of 6-fluoro-L-DOPA imaging agent for diagnosis of neuroendocrine tumors and Parkinson's disease", INTERNATIONAL JOURNAL OF PHARMACEUTICS, ELSEVIER, NL, vol. 519, no. 1-2, 1 January 2017 (2017-01-01), pages 304-313, XP009503920, ISSN: 0378-5173, DOI: 10.1016/J.IJPHARM.2017.01.038**
- **ISHIWATA K ET AL: "Electrophilic synthesis of 6-[<18>F]fluoro-l-dopa: Use of 4-O-pivaloyl-l-dopa as a suitable precursor for routine production", APPLIED RADIATION AND ISOTOPES, ELSEVIER, OXFORD, GB, vol. 44, no. 4, 1 April 1993 (1993-04-01), pages 755-759, XP024685346, ISSN: 0969-8043, DOI: 10.1016/0969-8043(93)90144-Y [retrieved on 1993-04-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 551 232 B1

SEQUENCE LISTING

<110> Itel Telecomunicazioni S.r.l. Università degli Studi di Bari Aldo Moro

<120> Stable F-DOPA formulations and uses thereof

<130> BWO19875-CF

<160> 3

<170> PatentIn version 3.5

<210> 1
<211> 24
<212> DNA
<213> artificial

<220>
<223> forward primer

<400> 1
ccagatcatg tttgagacct tcaa 24

<210> 2
<211> 22
<212> DNA
<213> artificial

<220>
<223> reverse primer

<400> 2
catacaggga cagcacagcc ty 22

<210> 3
<211> 18
<212> DNA
<213> artificial

<220>
<223> probe

<400> 3
accccagcca tgtacgta 18

## Description

### FIELD OF THE INVENTION

The present invention concerns the development of novel stable F-DOPA formulations and uses thereof.

### BACKGROUND OF THE INVENTION

Since the discovery in the 1980s that 6-F-dopa (F-DOPA) acts like an adrenergic false neurotransmitter, the use of 3,4-dihydroxy-6-[18F]-fluoro-L-phenylalanine ([$^{18}$F]F-DOPA) in positron emission tomographic (PET) imaging of human brain dopamine neurons, and more recently of neuroendocrine tumors (NETs) and glioma tissue, has been probed. Historically, PET studies were firstly concentrated on the brain and heart, while currently they are also useful imaging tools in oncology. This development derived from the use of [$^{18}$F]fluorodeoxyglucose (FDG) to face clinical problems related to human cancers. However, PET with FDG is not a suitable medical imaging approach for the diagnosis, staging and follow-up of well-differentiated and low aggressive malignancies. This deficiency was remedied using PET with the radiopharmaceutical containing [$^{18}$F]F-DOPA as a radiotracer, which ultimately provided the diagnosis of tumors, such as foci in the liver, pancreas or brain area, where enhanced uptake and decarboxylation of DOPA represents the diagnostic target.

The [$^{18}$F]F-DOPA formulations are used to study the striatal dopaminergic system physiopathology by PET-CT imaging and to visualize the regional distribution of the neurotransmitter in the human brain (e.g., in the diagnosis Parkinson's disease). More recently, the use of [$^{18}$F]F-DOPA in oncology, for NET and brain tumors visualization, was carried out with very promising perspectives. [$^{18}$F]F-DOPA use to detect NET by PET-CT imaging is due to the capability of these cells/tissues to up-take and store amino acids, including F-DOPA, through the neutral amino acid transporter expressed on their membrane.

NET are a complex group of neoplasms, arising from cells of the endocrine and nervous systems. The prognosis for patients with these pathologies is strictly connected to the location of the tumor and degree of differentiation. Improved diagnostic accuracy can be achieved by advanced imaging methods such as somatostatin receptor scintigraphy using SPECT, where the $^{111}$In-pentetreotide or other somatostatin analogues labeled with $^{99m}$Tc can be used because some NET over-express somatostatin receptors. A notable step forward in the diagnosis of these tumors occurred since the introduction of [$^{18}$F]F-DOPA in PET and its combination with computed tomography CT which is able to visualize a variety of neuroendocrine tumors. [$^{18}$F]F-DOPA appears to be superior in diagnostic performance in number of precise NET types such as medullar thyroid cancer, catecholamine-producing tumors with a low aggressiveness and well-differentiated carcinoid tumors of the midgut, and in cases of congenital hyperinsulinism.

IASOdopa® (IASON GmbH) (IASOdopa® SPC, 2016) and Dopacis® (CIS Bio International, IBA Molecular) (Dopacis® SPC, 2013) are two commercially available formulations of [$^{18}$F]F-DOPA, consisting in a solution for injection with activity per vial ranging from 0.15 GBq to 6.0 GBq at the date and time of calibration (half-life of the [$^{18}$P]-radionuclide = 110 min; Summary of Product Characteristics, 2006). However, they have some limitations. IASOdopa® solution for intravenous administration is prepared with the active ingredient [$^{18}$F]F-DOPA (1.05 mg/ml) mixed with acetic acid/sodium acetate buffer (pH = 2.3-3.0) through water for injections. Before the administration to a patient, the formulation pH must be adjusted to 4.0-5.0 by adding a sterile solution of $NaHCO_3$ (8.4 g/100 ml). Thereafter, the product should be stored below 25 °C for no longer than 2 h, and preserved from oxygen and light exposure (Summary of Product Characteristics, 2006) to avoid the radiotracer degradation through non-enzymatic auto-oxidation reactions. Hence, degradation of [$^{18}$F]F-DOPA may occur through oxidation of the catechol moiety mainly promoted by temperature above 25 °C, solution pH, $O_2$ and light exposure. Substituted aromatics phenols are susceptible of hydrogen abstraction, because aryls can stabilize the resulting radicals as in the case of the hydroquinone oxidation to quinone, which involves two one-electron transfer steps. Even though well-documented pharmaceutical active ingredient examples of catechol oxidation have been investigated, such as degradation of adrenaline to adrenochrome or the oxidation of apomorphine to oxoapomorphine, to our best knowledge not much is known, yet on the specific degradation of [$^{18}$F]F-DOPA.

It seems that significant infusion adverse site reactions, such as transient burning sensation and pain, observed following IASOdopa® i.v. administration in humans are partially attributed to the properties of the final formulation ([$^{18}$F]F-DOPA

solubility and instability, pH and osmolarity; Dopacis® SPC, 2013). Document US2012/029085 discloses a stable pharmaceutical formulation, comprising:

(a) a first composition that comprises the present compound fluorodopa, selected from a list;
(b) a second composition that comprises at least one local anesthetic compound, (c) at least one thiol agent; and
(d) at least one pH buffer that maintains a substantially constant pH in the pharmaceutical formulation,

wherein the pH is greater than about pH 5.5.

## Summary of the invention

The object of this disclosure is to provide novel [$^{18}$F]F-DOPA formulations, which do not present the drawbacks of the known [$^{18}$F]F-DOPA formulations. According to the invention, the above object is achieved thanks to the subject matter recalled specifically in the ensuing claims, which are understood as forming an integral part of this disclosure.

The present invention provides novel pharmaceutical formulations according to the claims comprising 6[$^{18}$F]-L-dihydroxyphenylalanine ([$^{18}$F]F-DOPA) and at least one buffering agent in a aqueous vehicle, wherein the formulation has a pH value of about 5.

Such new [$^{18}$F]F-DOPA preparations are easily adaptable to a fully automated synthesis module by using common pharmaceutical excipients, selected to guarantee the chemical stability of [$^{18}$F]F-DOPA in solution at a pH value compatible with the direct infusion avoiding the pain and burning at the injection site.

The present invention also concerns diagnostic uses of the novel [$^{18}$F]F-DOPA formulations herein disclosed.

## Brief description of the drawings

The invention will now be described in detail, purely by way of illustrative examples, with reference to the attached figures, wherein:

- **Figure 1. Decrease of F-DOPA peak area (o) as a function of time.** F-DOPA is dissolved in phosphate buffer (0.05 M) at pH 8.0 at 25 °C.
- **Figure 2. FT-IR monitoring of the F-DOPA degradation at pH 5.15 and 8.0.**
- **Figure 3. The pH-rate profile of F-DOPA at 25 °C.** In the upper scheme, the acid-base equilibria of F-DOPA are reported, along with the p$K_a$ values calculated with the ACDLabs software.
- **Figure 4. Novel F-DOPA formulations (ND1 and ND2) concentrationmortality rate of mouse skeletal muscle fibers, renal tsA and neuronal SH-SY5Y cells after 3 h of incubation time.** A) Skeletal muscle fiber cell mortality was directly determined by counting enzymatically isolated fibers from *Flexor Digitorum Brevis* (FDB) muscle before and after the incubation with ND1 or ND2. (B) Neuronal SH-SY5Y and (C) renal tsA cell mortality assay was performed by Scepter™ 2.0 Cell Counter Merck/Millipore. Each data point represents the mean ± SEM of at least three replicates. The concentration-response relationships of the ND2 were shifted to the right on the log concentration axis with respect to that of the ND1 and ND3 in all cell types.
- **Figure 5. Local reactions of male Wistar rat *Tibialis Anterior* muscle after 30 min. following the i.m. administration of ND2, ND3 and their respective vehicles.** A) The i.m. injection of 0.2 ml of acetic acid-based ND3 formulation at the concentration of 10 mg/ml (5 mg/kg) caused a marked redness and darkening of the left hand muscle. B) No local reaction was observed after intramuscular injection of lactate/$Na_2$EDTA-based ND2 formulation (5 mg/kg). The administration of either acetic acid-based VD3 (C) and lactate/$Na_2$EDTA-based VD2 (D) did not cause visual local reaction in the right hand muscles of the same rat. Representative sections of *Tibialis Anterior* (TA) muscle after 30 minutes from the local intramuscular injection into the same muscle were reported. E) Control, physiological solution, F) ND2 and G) ND3. Perimisial vacuoles were observed in the muscle sections from controls, ND2 and ND3 sections. Cytosolic opalescent areas, representative of death fibers, were particularly observed in the ND3 muscle section. These fibers also showed a reduced cross sectional area.
- **Figure 6. Gene expression profile of *Tibialis Anterior* muscle after 30 min. following the i.m. administration of F-DOPA formulations and their respective vehicles in male Wistar rats.** The experimental groups were: CTRL, controls rats treated with physiological solution; ND3, rats treated with acetic acid based F-DOPA (5 mg/kg); ND2, rats treated with lactate/$Na_2$EDTA based F-DOPA (5 mg/kg); VD3, rats treated with the acetic acid vehicle solution; VD2, rats treated with lactate/$Na_2$EDTA vehicle solution. Each bar represents the mean ± S.E.M. of at least three samples. One way ANOVA analysis showed that the expression level of *Pgc1-alpha, Lc3, Cgrp, Casp3, 8, 9, Mapk3* and *Tnf-alpha* genes was significantly enhanced following treatments with ND3 vs CTRL controls ($p<0.05$). The *Casp3, 8, 9, Lc3, Bnip3, Pgc1alpha, Mapk3, Tnf-alpha* and *Cgrp* genes were up-regulated following VD3 treatment vs CTRL ($p<0.05$). The ND2 treatment significantly enhanced the expression levels of the *Mapk3* and *Pgc1alpha*genes ($p<0.05$).
- **Figure 7. Percent change (%) of body weight following the i.v. administration of a single dose of F-DOPA formulations after a follow-up of 14 days in male Wistar rats.** The experimental groups were: CTRL, rats treated with physiological solution (N rats = 3); VD2, rats treated with lactate/$Na_2$EDTA vehicle solution (N rats = 3); ND2, rats treated with lactate/$Na_2$EDTA based F-DOPA (0.025 mg/kg) (N rats = 3) and ND2 (5 mg/kg) (N rats = 3); VD3, rats treated with the acetic acid vehicle solution (N rats = 3); ND3, rats treated with acetic acid based F-DOPA (0.025 mg/kg) (N rats = 3) and ND3 (5 mg/kg) (N rats = 3). Each bar represents the mean ± S.E.M. of three animals. The

statistical analysis performed with one way ANOVA showed a significant difference among groups which was determined by the ND3 (5 mg/kg) group that caused a mild drop of body weight ($p<0.05$).

- **Figure 8. Changes of organs wet weight normalized to body weight following the i.v. administration of a single dose of F-DOPA** formulations **after a -up of 14 days in male Wistar rats.** The experimental groups were: CTRL, rats treated with physiological solution (N rats = 3); VD2, rats treated with lactate/$Na_2$EDTA vehicle solution (N rats = 3); ND2, rats treated with lactate/$Na_2$EDTA based F-DOPA (0.025 mg/kg) (N rats = 3) and ND2 (5 mg/kg) (N rats = 3); VD3, rats treated with the acetic acid vehicle solution (N rats = 3); ND3, rats treated with acetic acid based F-DOPA (0.025 mg/kg) (N rats = 3) and ND3 (5 mg/kg) (N rats = 3). The organs were: *Tibialis Anterior* (TA), *Extensor Digitorum Longus* (EDL) and *Soleus* (SOL) muscles; heart, brain, liver and kidneys (right and left). Each bar represents the mean ± S.E.M. of at least three samples. The statistical analysis performed with one way ANOVA showed a not significant difference among groups.
- **Figure 9. Gene expression profiles of whole brain and *Tibialis Anterior* (TA) muscle following the i.v. administration of a single dose of F-DOPA formulations and their respective vehicles after a follow-up of 14 days in male Wistar rats.** The experimental groups were from left to right: CTRL, control rats treated with physiological solution; VD3, rats treated with the acetic acid based vehicle solution; ND3, rats treated with acetic acid based F-DOPA (5 mg/kg); VD2, rats treated with lactate/$Na_2$EDTA based vehicle; ND2, rats treated with lactate/$Na_2$EDTA based F-DOPA (5 mg/kg). Each bar represents the mean ± S.E.M. of at least three samples. One way ANOVA analysis showed that the expression level of *Pgc1-alpha* and *Cgrp* genes was significantly enhanced following ND3 treatment and a follow-up of 14 days in rat brain vs controls ($p<0.05$). The *Cgrp* gene was also up-regulated in the VD3 vehicle group ($p<0.05$). No changes of the gene expression following i.v. treatment with the F-DOPA formulations were observed in the muscle of the same rats.
- **Figure 10. Percent change (%) of body weight following the i.v. administration of a single dose of F-DOPA formulations after a follow-up of 14 days in male C57BL/6J mice.** The experimental groups were: CTRL, mice treated with physiological solution (N mice = 4); VD2, mice treated with lactate/$Na_2$EDTA vehicle (N mice = 4), ND2, mice treated with lactate/$Na_2$EDTA based F-DOPA (50 mg/kg) (N mice = 4); VD3, mice treated with the acetic acid based vehicle solution (N mice = 4); ND3, mice treated with acetic acid based F-DOPA (50 mg/kg) (N mice = 4). The statistical analysis performed with one way ANOVA showed a significant difference among groups. This difference was determined by the ND3 group and VD3 groups that reduced body weight after 14 days of follow-up ($p<0.05$).
- **Figure 11. Change of organs wet weight normalized to body weight following the i.v. administration of a single dose of F-DOPA formulations after a follow-up of 14 days in male C57BL/6J mice.** The experimental groups were: CTRL, mice treated with physiological solution (N mice = 4); VD2, mice treated with lactate/$Na_2$EDTA vehicle (N mice = 4), ND2, mice treated with lactate/$Na_2$EDTA based F-DOPA (50 mg/kg) (N mice = 4); VD3, mice treated with the acetic acid based vehicle solution (N mice = 4); ND3, mice treated with acetic acid based F-DOPA (50 mg/kg) (N mice = 4). The organs were: *Tibialis Anterior* (TA), *Extensor Digitorum Longus* (EDL) and *Soleus* (SOL) muscles; heart, brain, lung, liver and kidneys (right and left). Each bar represents the mean ± S.E.M. of at least four samples. The statistical analysis performed with one way ANOVA showed a not significant difference among groups after 14 days of follow-up.
- **Figure 12. Gene expression profile of whole brain and *Tibialis Anterior (TA)* muscle following the i.v. administration of a single dose of F-DOPA formulations after a follow-up of 14 days in male C57BL/6J mice.** The experimental groups were from left to right: CTRL, control mice treated with physiological solution (N mice = 4); VD3, mice treated with the acetic acid based vehicle solution (N mice = 4); ND3, mice treated with acetic acid based F-DOPA (50 mg/kg) (N mice = 4); VD2, mice treated with lactate/$Na_2$EDTA based vehicle (N mice = 4); ND2, mice treated with lactate/$Na_2$EDTA based F-DOPA (50 mg/kg) (N mice = 4). Each bar represents the mean ± S.E.M. of at least four samples. The *Lc3* and *Bnip3* genes were up-regulated following ND3 (50 mg/kg) and VD3 treatments in *Tibialis Anterior* muscle (One way ANOVA $p<0.05$). The *Lc3* gene was also significantly up-regulated following the ND2 (50 mg/kg) treatment ($p<0.05$). In contrast, the expression levels of *Atrogin-1, Murf-1, Pgcl alpha, UCP1, Nfkb1, Casp3,* 9 and *Tnf alpha* genes were undetectable in *Tibialis Anterior* muscle.

## DESCRIPTION OF THE INVENTION

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures,

or characteristics may be combined in any suitable manner in one or more embodiments.

The headings provided herein are for convenience only and do not interpret the scope or meaning of the embodiments.

The present invention concerns a novel pharmaceutical formulation as defined in the claims comprising 6-[$^{18}$F]-L-dihydroxyphenylalanine ([18F]F-DOPA), at least one metal cation chelating agent and at least one buffering agent in a aqueous vehicle, wherein the buffering agent is lactate, wherein the formulation has a pH value of about 5.

In one embodiment, the formulation comprises at least one of: at least one ionic force regulator agent, at least one preservative agent, at least one isotonic agent.

In a further embodiment, the at least one metal cation chelating agent is selected from: EDTA, citric acid, tartaric acid, and salts thereof.

In a further embodiment, the at least one preservative agent is selected from sulfurated compounds, preferably sodium bisulfite and sodium sulfite, ascorbic acid and boric acid and salts thereof.

In one embodiment, the at least one ionic force regulator agent is sodium chloride. In one embodiment, the at least one ionic force regulator agent is present in any amount to provide the formulation with an ionic force less or equal to 0.150 M.

In one embodiment, the formulation has a not controlled ionic force.

In a further embodiment, the formulation is isotonic.

In a further embodiment, the at least one isotonic agent is selected from sodium chloride, glucose.

In one embodiment, the aqueous vehicle is substantially free of oxygen species, preferably the aqueous vehicle is deoxygenated water for pharmaceutical use, more preferably is deoxygenated double distilled water.

According to one embodiment, the pharmaceutical formulation herein disclosed is for use as a radiotracer in an imaging diagnostic technique, preferably a positron emission tomography (PET).

According to one embodiment, the pharmaceutical formulation herein disclosed is for use in diagnosis of neurodegenerative diseases, preferably Parkinson's disease. According to one embodiment, the pharmaceutical formulation herein disclosed is for use in diagnosis of tumors, preferably neuroendocrine tumors (NETs). According to one embodiment, the pharmaceutical formulation herein disclosed is for use in diagnosis of a tumor in a subject, wherein the tumor is preferably selected from: brain tumors, pancreas tumor, medullary thyroid cancer, catecholamine-producing tumors, carcinoid tumors of midgut, ovarian cancer, sentinel lymph nodes.

In present description further discloses a method for diagnosing neurologic disorders and neurodegenerative diseases or tumors in a human subject by an imaging diagnostic technique, comprising:

- intravenously administering to the subject a pharmaceutical formulation comprising 6-[$^{18}$F]-L-dihydroxyphenylalanine and at least one buffering agent in an aqueous vehicle, wherein the formulation has a pH value comprised between 4.5 and 5.5, preferably about 5;
- allowing a target tissue to uptake 6-[$^{18}$F]-L-dihydroxyphenylalanine; and
- obtaining an image of the target tissue.

Oxidation reactions represent a crucial degradation pathway of many pharmaceutical active principle ingredients. Unfortunately, only few detailed studies have been performed on the oxidative degradation of F-DOPA, hence the use of antioxidants and excipients modulating the solution pH in its pharmaceutical formulations remains largely empirical. F-DOPA degradation mainly occurs through oxidation of the catechol moiety that is a reaction involving two one-electron transfer steps. The first electron transfer is usually catalyzed by transition metals, yielding the semiquinone radical anion; then it transfers an electron to molecular oxygen forming superoxide and the corresponding quinone. Although, the catechol oxidation has been well investigated, e.g., degradation of adrenaline to adrenochrome and oxidation of apomorphine to oxoapomorphine, to our best knowledge, very few studies have been reported so far on the specific degradation of F-DOPA. In order to independently confirm the expected degradation pathway (Scheme 1), the stability of F-DOPA was initially monitored in phosphate buffer (0.05 M) at pH 8.0, 25 °C. A plot of the disappearance of F-DOPA and the appearance of the degradation product as a function of time is shown in Figure 1. These results are consistent with the observations that, qualitatively, the kinetics of oxidative free-radical reactions follows a pattern characterized by a lag phase corresponding to the gradual buildup of radicals *via* an initiation step, followed by rapid loss of the drug approximating first order kinetics. The overall kinetics cannot be defined as a first order process. However, the oxidation kinetics is further complicated when the state of ionization of the molecule is affected by the solution pH. Kinetics and mechanism of oxidation of phenols and *ortho*- and *para*-dihydroxybenzenes in aqueous solution are very complex and pH-dependent. Therefore, pH-rate profiling was used to further characterize the degradation pathway and mechanism.

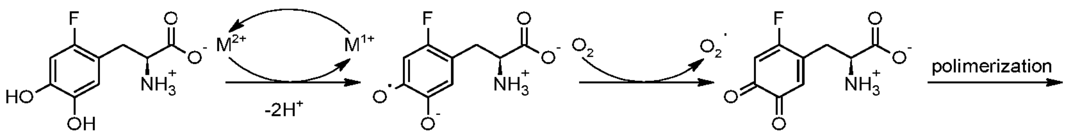


**Scheme 1.** Exemplified proposed degradation pathway for F-DOPA.

Aimed at better understanding the nature of the F-DOPA degradation products, the progress of the oxidation of F-DOPA was investigated by *in situ* FT-IR experiments at pH 8.0 and 5.15 at 25 °C (Figure 3). A broad signal in the range 1660-1640 $cm^{-1}$ was observed for the F-DOPA at pH 5.15 that was assigned to $v_{C=O}$. After 16 h, at pH 5.15, no additional peaks in the range 1800-1200 $cm^{-1}$ were detected thus demonstrating, the stability of F-DOPA in acidic solutions.

In a second experiment, using a buffer at pH = 8.0, the *in situ* IR analysis of the reaction showed a slow degradation profile (10% loss of $v_{C=O}$ absorbance within 2 h) and new signals at 1510 and 1405 $cm^{-1}$ were observed most likely related to C=C stretching of oxidized products. Nevertheless, an overlapping of the carbonyl and carboxyl groups in the range 1670-1650 $cm^{-1}$ of the postulated o-quinone derivatives (Scheme 1) should occur in aqueous solution. However, similar o-quinone species show typical bands at 1660, 1480 and 1380 $cm^{-1}$, and are known to be very unstable turning black the solution upon standing. Also in our case, after stirring at pH = 8.0 for 24 h, the transparent aqueous solution of F-DOPA turned to black (Figure 2) as a consequence of a degradation process. Thus, *in situ* IR experiments confirm the long-time stability of F-DOPA in acidic conditions (pH < 5.15) and the high propensity to undergo degradation under basic conditions. The pH-rate profile was carried out in order to obtain kinetic data.

*pH-rate profile*

The kinetics of the degradation of F-DOPA were investigated in aqueous solution at various pH values in the range 1-8 at 25 °C (ionic strength 0.15 M adjusted by adding NaCl). Since the kinetics of F-DOPA loss cannot be described by first order kinetics, the pH-rate profile is reported here as Log $1/t_{90\%}$ versus pH (Figure 3). The results suggested that the degradation of F-DOPA has a complex pH dependency and becomes base dependent above pH 7.0. The shelf life for F-DOPA degradation at 25 °C was determined at various pH values ranging from 1 to 8 and was used to construct the pH-rate profile, whose shape suggests that the anionic form of the F-DOPA catechol is the most sensitive to oxidation at pH values greater than 7.0. Below pH = 7.0, the pH-rate profile follows a complex pH dependency. This experimental evidence is in agreement with the calculated $pK_a$ values (ACDLabs software, $pK_a$ module, Figure 3). As shown in Figure 3, F-DOPA, as L-DOPA ($pK_{a1}$ = 2.24, $pK_{a2}$ = 9.30, $pK_{a3}$ = 9.87, $pK_{a4}$ = 12.62) is characterized by four ionizable groups and consequently by four $pK_a$ values ($pK_{a1}$ = 2.24 $\pm$ 0.14, $pK_{a2}$ = 8.70 $\pm$ 0.33, $pK_{a3}$ = 8.95 $\pm$ 0.25, $pK_{a4}$ = 12.64 $\pm$ 0.23), which regulate the existence domain of each entity of F-DOPA in function of the pH. At pH values above 7.0 the catechol moiety of F-DOPA is susceptible to hydrogen abstraction leading to the anionic species $H_2L$, HL and L that are the most sensitive to oxidation.

This behavior was further supported by monitoring the change in UV-Vis absorbance of a pH 6.4 sterile physiological solution containing F-DOPA. Three F-DOPA solutions, at the final concentrations ranging from 1 to 4 mg/mL in a degassed perfusional sterile 0.9% NaCl solution, were incubated at 25 or 45 °C and their absorbance at $\lambda$ = 298 nm was recorded for 6 h. During this time only a slight change in absorbance was observed, by showing less than 5% decrease in F-DOPA initial concentration, and only after 24 h incubation a pale pink color in each sample appeared, as a consequence of a slow oxidation, as assessed by the slight increased absorbance at $\lambda$ = 495 nm.

In addition, enantioselective HPLC analysis, carried out using the chiral CHIROBIOTIC TAG stationary phase, revealed that, like in L-DOPA, no racemization occurs in F-DOPA (4 mg/mL) incubated for 24 h at both 25 and 45 °C in a sterile perfusion solution at pH 6.0.

*Preparation of F-DOPA formulations*

Optimum stability for F-DOPA in aqueous formulations is achieved by maintaining the pH at acidic values, by excluding oxygen and incorporating in the formula a chelating agent for metal ion initiators of free-radical oxidation reactions. Hence, the final formulations using deoxygenated double-distilled water were in glass vials under an inert atmosphere and stored in the dark. In order to formulate F-DOPA at the optimum acidic pH range, the appropriate buffer was selected. Lactate buffer (1 mM, pH 5.0) was chosen to formulate the parenteral solution, firstly because lactic acid is currently used in intravenous solutions and is already present in commercial formulation and, secondly, because this solution has a pH value compatible with the direct infusion avoiding or minimizing the pain at the injection site. Finally,

common methods for decelerating the initiation step require eliminating initiators and catalysts from the formulation. Metals ions can be sequestered by the use of chelators, such as EDTA, citric acid and tartaric acid. In a previous study, the addition of $Na_2EDTA$ (0.15%) effectively proved to retard the auto-oxidation rate of F-DOPA. Hence, as reported in Table 1, two parenteral formulations of F-DOPA, namely **ND1** and **ND2,** were prepared using 1 mM lactate buffer (pH 5.0) aqueous solution in the presence of 1 mM $Na_2EDTA$ with or without ionic strength adjusted to 150 mM by the addition of NaCl. For comparison purposes the **ND3** formulation, having the same commercially available formulation "master formula", was also prepared by solubilizing F-DOPA in an aqueous solution of 17.5 mM acetic acid (1.05 mg/mL, pH = 3.1). In order to mimic the commercial formulation, immediately before its use the pH of **ND3** was adjusted to a higher pH value (measured pH = 7.2) by the addition of 100 μL per mL of a sterile solution of 1 M (84 mg/mL) sodium bicarbonate. These compositions allowed to dissolve 10 mg of F-DOPA per mL.

**Table 1.** Composition of the F-DOPA formulations.

| **Formulation** | **Composition** | | | |
|---|---|---|---|---|
| | Buffer (conc.) | $Na_2EDTA$ (mM) | pH | Ionic strength (mM)[a] |
| **ND1** | Lactate (1 mM) | 1 | 5.0 | 150 |
| **ND2** | Lactate (1 mM) | 1 | 5.0 | - |
| **ND3**[b] | Acetic acid (17 mM) | - | 3.0 | - |

[a]The ionic strength was adjusted to 150 mM by the addition of NaCl.
[b]Before its use the pH was adjusted by the addition of 100 μL per mL of a sterile solution of sodium bicarbonate (84 mg/mL).

*Chemical stability studies of F-DOPA in the parenteral formulations*

The commercial formulation of [$^{18}$F]F-DOPA has a declared shelf life ($t_{90\%}$) of 12 h from the time of calibration and 8 h after first withdrawal (Summary of Product Characteristics, 2006). After pH-adjustment with 84 mg/mL sodium bicarbonate solution, the product should be stored below 25 °C for no longer than 2 h (Summary of Product Characteristics, 2006). Stability studies of F-DOPA in the new formulations **ND1** and **ND2,** designed with the purpose to avoid the pH adjustment before injection, were conducted at 25 and 40 °C. The results showed that F-DOPA in the solutions **ND1** and **ND2** was stable for more than 12 h at those temperatures. F-DOPA in **ND1** formulation has a shelf life ($t_{90\%}$) of 37.5 and 34.2 h at 25 and 40 °C, respectively, whereas in **ND2** formulation F-DOPA has a shelf life ($t_{90\%}$) of 54.8 and 51.0 h at 25 and 40 °C, respectively (Table 2). These results once more suggest that the stability of formulated F-DOPA is related to factors involved in the oxidation of the catechol group, such as the degree of metal contamination, oxygen concentration and pH, but also to the temperature, which affects the amount of dissolved oxygen and the pH value. In addition, the shelf life values show that the loss of F-DOPA in formulation **ND2** is significantly better than that observed for formulation **ND1.** This might be due to the lower ionic strength.

**Table 2.** Shelf life for the F-DOPA degradation in **ND1** and **ND2** formulations at 25 and 40 °C.

| **Formulation** | **T (°C)** | **$t_{90\%}$ (h)**[a] |
|---|---|---|
| **ND1** | 25 | 37.5 |
| | 40 | 34.2 |
| **ND2** | 25 | 54.8 |
| | 40 | 51.0 |

[a]Data are means of three determinations. Each experiment was performed in triplicate.

*Cyclic temperature stress test*

All formulations of F-DOPA were stable when stored in the dark for a total of 60 h, 12 h for each step, in a climatic chamber at temperature and RH values of 4 ± 2 °C, 25 ± 2 °C/60 ± 5% RH, 40 ± 2 °C/60 ± 5% RH, 25 ± 2 °C/60 ± 5% RH, and 4 ± 2 °C. The physical stability study showed that the **ND1** and **ND2** formulations remained unchanged with respect to color, and no turbidity or precipitate formation was detected in the storage conditions. The pH value (5.0) was also stable and the percentage of F-DOPA remaining with respect to initial content was found to be 92.1 ± 0.7% and 98.5 ± 0.3% for formulation **ND1** and **ND2,** respectively. Formulation **ND2** showed no change in content and chromatographic purity during the storage condition, while formulation **ND1** proved to be the least stable

formulation, but still with a percentage of F-DOPA above 90% after 60 h of incubation.

*"In vitro " experiments: cell viability assays.*

The "in vitro" incubation of the mouse skeletal muscle fibers for 3 h with 0.02 mg/ml concentration of the F-DOPA, **ND1, ND2** and **ND3** showed that there were no significant differences among the formulations under investigations with cell mortality < 15%. At the active ingredient concentration of 0.05 mg/ml, F-DOPA and **ND3** induced a cell mortality rate > 50% and significantly higher than that of **ND1** and **ND2** formulations as determined by one way analysis of variance (ANOVA) and Bonferroni test ($p < 0.05$). At the active ingredient concentration of the 0.2 mg/ml all formulations induced a cell mortality of 100%. Therefore, **ND1** and **ND2** were better tolerated than **ND3** and F-DOPA in this cell type (Figure 4A).

In human neuroblastoma dopaminergic SH-SY5H cell line, the mortality rate caused by the **ND3** and **ND1** formulations *vs* **ND2** was significantly enhanced at 0.05 mg/ml active ingredient concentration as determined by one way analysis of variance (ANOVA) and Bonferroni test ($p < 0.05$). At the active ingredient concentration of the 0.2 mg/ml, **ND1** induced a cell mortality > 75%. On SHSY5H cells, **ND2** was better tolerated than **ND3** and **ND1** formulations (Figure 4B).

Human renal tsA201 cell line mortality rate in the presence of **ND1** and **ND2** formulations *vs* ND3 was significantly enhanced at 0.005 mg/ml and 0.05 mg/ml active ingredient concentrations as determined by one way analysis of variance (ANOVA) and Bonferroni test ($p < 0.05$). At the concentration of the 0.2 mg/ml, all formulations induced a comparable cell mortality > 75%. On tsA201 cells, **ND3** was better tolerated than **ND2** and **ND1** formulations (Figure 4C). A significant difference in terms of cell mortality rate between formulations in all cell types was observed at 0.05 mg/ml concentration.

The concentration-response relationships for these drugs revealed that the $EC_{50}$ and $E_{max}$ calculated by fitting routine were favorable to **ND2** formulation *vs* **ND1** and **ND3** formulations in the different cell lines (Table 3). The rank order of potency in inducing cell mortality based on $EC_{50}$ values was: F-DOPA ≥ **ND3** > **ND1** ≥ **ND2** in skeletal muscle fibers, **ND3** > **ND1** > **ND2** in SH-SY5H cells and **ND1** > **ND3** > **ND2** in tsa201 cells.

Table 3. Fitting parameters of the concentration-response relationships of F-DOPA formulations in different cell types.

| Formulations | Skeletal muscle fibers | | | SHSY5Y cells | | | tsa201 cells | | |
|---|---|---|---|---|---|---|---|---|---|
| | $EC_{50}$ (M) | n | $E_{max}$ (%) | $EC_{50}$ (M) | n | $E_{max}$ (%) | $EC_{50}$ (M) | n | $E_{max}$ (%) |
| **F-DOPA** | $1.85 \times 10^{-4}$ | 15.06 | 100 | | | | | | |
| **ND1** | $2.70 \times 10^{-4}$ | 2.76 | 100 | $2.43 \times 10^{-4}$ | 5.42 | 80 | $1.34 \times 10^{-4}$ | 0.65 | 100 |
| **ND2** | $2.73 \times 10^{-4}$ | 4.50 | 100 | $6.32 \times 10^{-4}$ | 9.76 | 50 | $3.99 \times 10^{-4}$ | 1.57 | 100 |
| **ND3** | $1.89 \times 10^{-4}$ | 3.88 | 100 | $2.19 \times 10^{-4}$ | 9.26 | 60 | $2.56 \times 10^{-4}$ | 1.28 | 100 |

$E_{max}$ = maximal mortality rate; n = slope of the curve; $EC_{50}$ = concentration needed to induce the 50% of mortality rate.

*"In vivo " experiments: local tolerability test in rat.*

The i.m. injection of the **ND3** formulation (5 mg/kg) (0.2 ml injected volume) into the left hand *Tibialis Anterior* muscle of conscious rats (N° of rats = 3) caused, in all treated animals, a local reaction consisting of marked redness and darkening of the injected area and a mild oedema formation within 30 minutes from the administration, clinically reversible after 4h from the administration (Figure 5A). In contrast, the rats treated with **ND2** formulation (5 mg/kg) (N° of rats = 3) did not show any sign of local reaction within the 24h from the administration (Figure 5B). No visual local reaction was observed on the right rat muscles treated with the VD3 and VD2, vehicles of **ND3** and **ND2,** respectively, (N° of rats = 3) (Figure 5C and 5D). The i.m. treatment of the rats with low dose of **ND3** (N° of rats = 3) (0.1 mg/kg) and **ND2** (0.1 mg/kg) (N° of rats = 3) as well as with physiological solution did not produce any reaction.

No clinically significant animal reactions to hindpaw, i.p. and s.c. injections of the formulations were observed in the rats at the same dosing schedule (0.1 mg/kg - 5 mg/kg) in terms of hindlimb licking, shaking and withdrawal, convulsion, piloerections and oedema formation within 24h following injection.

Histological and RT-PCR investigations were performed in *Tibialis Anterior* muscle (muscle wet weight in controls = 520.5 ± 12 mg, N° = 3 muscles) collected from rats after 30 minutes from the i.m. treatment with physiological solution (N° muscles/rats = 3/3), **ND2** (5 mg/kg) (N° muscles/rats = 3/3) and **ND3** (5 mg/kg) (N° muscles/rats = 3/3) formulations, VD2 (N° muscles/rats = 3/3) and VD3 (N° muscles/rats = 3/3). The histological analysis of *Tibialis Anterior*

muscle sections revealed the presence of an elevated number of death fibers (Number of sections per muscle = 10) from rats treated intramuscularly with the **ND3** (5 mg/kg) and VD3 formulations which was significantly different *vs* controls, VD2 (5 mg/kg) and **ND2** rat groups as determined by student t-test ($p < 0.05$). In addition, this parameter was significantly elevated in **ND3** *vs* VD3 vehicle ($p < 0.05$) (Table 4). The death fibers of the **ND3** group showed also a significantly reduced cross section area (CSA) as compared to that measured in the normal fibers ($p<0.05$). Perimysium vacuoles were observed in the muscle sections from controls and **ND2** and **ND3** (Figure 5E, 5F and 5G).

**Table 4.** Number of death fibers and cross sectional area (CSA) in *Tibialis Anterior* muscle after 30 min following the i.m. administration of F-DOPA formulations in male Wistar rat.

| Treatments | Death fibers | Normal fibers |
|---|---|---|
| VD3 vehicle (N of sections =10) | N of fibers = 57+16 *Δ CSA = 1620 ± 119 $\mu m^2$ | N of fibers = 47±11 * CSA = 1930 + 221 $\mu m^2$ |
| **ND3** (5 mg/kg) (N of sections=10) | N of fibers = 97+9 *Δ□ CSA = 1610 ± 112 $\mu m^2$ | N of fibers = 23+8 * CSA = 1970 + 210 $\mu m^2$ |
| VD2 vehicle (N of sections =10) | N of fibers = 31+7 ° CSA = 1750 ± 210 $\mu m^2$ | N of fibers = 93+13 CSA = 1930 + 111 $\mu m^2$ |
| **ND2** (5mg/kg) (N of sections =10) | N of fibers = 30+8° CSA = 1770 + 201 $\mu m^2$ | N of fibers = 95+9 CSA = 1970 + 231 $\mu m^2$ |
| CTRL (N of sections =10) | N of fibers = 13+3 CSA = 1770 + 234 $\mu m^2$ | N of fibers = 101±11 CSA = 2170 + 230 $\mu m^2$ |

The experimental groups were: CTRL, controls rats treated with physiological solution; ND3, rats treated with acetic acid based F-DOPA (5 mg/kg); ND2, rats treated with lactate/$Na_2EDTA$ based F-DOPA (5 mg/kg); VD3, rats treated with the acetic acid vehicle solution; VD2, rats treated with lactate/$Na_2EDTA$ vehicle solution. The data represents the mean ± S.D. of cross sectional area (CSA) and number of fibers from randomly selected muscle sections of the same area. Data significantly different: □ ND3 vs VD3 vehicle, * ND3 vs control (CTRL) treated with physiological solution, Δ ND3 vs VD2, °ND2 and VD2 groups vs CTRL ($p<0.05$) as determined by *student t test.*

RT-PCR experiments showed that the mRNA levels of *Pgc1-alpha, Lc3, Cgrp, MapK3, Tnf-alpha* and *Caspase 3, 8, 9* genes were significantly enhanced after 30 minutes from the i.m. **ND3** (10 mg/kg) administration into the same muscle, as determined by one way ANOVA ($F = 3.13$, $p < 0.05$) in the affected *Tibialis Anterior* muscles (Figure 6). This difference was determined for the **ND3** (10 mg/kg) group, as revealed by the Bonferroni and Tukey HSD tests (Tukey HSD *test p* values: ND3 *vs* CTRL, $p < 0.05$). While, the expression levels of *Atrogin1, Murf1, Bnip3, TrpV1, Caspase 3 and 9* genes were not significantly affected. In addition, the *Caspase 3, 8, 9, Lc3, Bnip3, Pgcl alpha, Mapk3, Tnf-alpha* and *Cgrp* genes were up-regulated following the VD3 treatment (Tukey HSD test *p* values: VD3 vs CTRL $p < 0.05$). The **ND2** (10 mg/kg) treatment significantly enhanced the expression levels of the *Mapk3* and *Pgcl alpha* genes ($p < 0.05$). No significant effect was observed on sampled genes with the VD2 (Figure 6).

*"In vivo " experiments: Single Dose Extended Toxicity study in rats and mice.* A *Single Dose Extended Toxicity* study was performed, in which the acute toxicity was evaluated following a single i.v. dose in rats and a follow-up period of 14 days to detect the reversibility of the observed effects. The animals were sacrificed and then all organs and blood collected for further analysis at the end of the follow-up (EMA/CHMP/CVMP/JEG-3Rs/169839/2011-Rev.1). The body weight change analysis performed comparing the changes of body weight of the rats at the end of follow-up *vs* the body weight before any treatment revealed a mild but significant difference among groups as determined by one way ANOVA ($F = 5.37$, $p < 0.005$). This difference was determined for **ND3** (5 mg/kg), as revealed by the Bonferroni and Tukey HSD tests [Tukey HSD test p values: **ND3** *vs* CTRL, $p < 0.005$; **ND3** (5 mg/kg) *vs* **ND2** (5 mg/kg), $p < 0.05$; ND3 (5 mg/kg) *vs* VD3, $p < 0.0051$] (Figure 7). No change of wet organs weight between groups (ANOVA one way, $F = 1.2$) was observed (Figure 8). Moreover, the metabolic and physiological parameters of the rats such as food and water consumption, urine and feces production were not significantly different among groups after 14 days of follow-up (ANOVA one way, $F = 1.1$) (Table 5).

The analysis carried out on intracardiac blood samples collected after 14 days of follow-up in rats did not reveal any significant changes, among experimental groups, in the serum levels of cardiac troponin C, aminotransferases ALT and AST, amylase, creatin kinase CK, creatinine and urea used as indices of heart, liver, pancreatic, skeletal muscle and renal damages (ANOVA one way, $F = 1.11$). No significant change in the serum TNFalpha, IL-6 and IL-1b levels among groups was observed after treatments (ANOVA one way, $F = 1.1$) (Table 6).

Table 5. Metabolic data following a single i.v. dose of F-DOPA formulations after 14 days of follow-up in rats and mice.

| Parameters | CTRL | VD2 | **ND2** (0.025 mg/kg in rat) | **ND2** (5 mg/kg in rat) (50 mg/kg in mice) | VD3 | **ND3** (0.025 mg/kg in rat) | **ND3** (5mg/kg in rat) (50 mg/kg in mice) |
|---|---|---|---|---|---|---|---|
| **Rat Food consumed (g)** | 24.67±4.6 | 24.67±4.8 | 20.33±0.33 | 19.33±1.3 | 27.33±2.7 | 16.00±3.5 | 15.33±2.91 |
| **Water consumed (ml)** | 37.50±12.5 | 23.67±2.3 | 32.33±7.9 | 31.67±11.7 | 25.67±6.9 | 24.33±2.9 | 20.67±5.20 |
| **Urine produced (ml)** | 16.33±5.8 | 16.33±0.9 | 14.00±3 | 24.33±10.6 | 17.00±1 | 14.33±0.7 | 14.33±2.33 |
| **Feces produced (g)** | 12.67±1.7 | 12.67±3.5 | 9.00±1.1 | 9.50±0.9 | 9.00±0.7 | 6.00±2 | 7.00±0.66 |
| **Mice Food consumed (g)** | 2.15±0.6 | 1.44±0.7 | - | 2.00±1.1 | 1.54±0.75 | - | 1.42±1.30 |
| **Water consumed (ml)** | 3.33±0.9 | 10.67±2.3 | - | 10.67±2.3 | 7.33±1.45 | - | 9±4 |
| **Urine produced (ml)** | 1.65±0.2 | 2.54±0.2 | - | 2.00±0.6 | 1.56±0.77 | - | 2.17±0.4 |
| **Feces produced (g)** | 0.75±0.1 | 0.65±0.2 | - | 0.93±0.3 | 0.83±0.29 | - | 0.655±0.41 |

The data were not significantly different among groups as determined by one way analysis of variance.

**Table 6.** Cytokines and biochemical markers following a single i.v. dose of F-DOPA formulations after 14 days of follow-up in rats.

| | **IL-1b pg/ml** | **IL-6 pg/ml** | **TNF-α pg/ml** | **Cardiac Troponin I ng/ml** | **ALT mg/dl** | **AMILASI mg/dl** | **AST mg/dl** | **CK mg/dl** | **CREA mg/dl** | **UREA mg/dl** |
|---|---|---|---|---|---|---|---|---|---|---|
| VD2 (N=3) | 27.5±1 | OOR < | OOR < | 2.7±0.5 | 42.0±1.63 | 1205.0±216.4 | 103.5±7 | 785.0±174 | 0.4±0.01 | 21.5±9.4 |
| ND2 (N=3) (0.025 mg/kg) | 39.49±4 | OOR< | OOR < | 0.784±0.02 | 54±3 | 1350±120 | 122±19 | 1130±123 | 0.36±0.03 | 35±5 |
| ND2 (N=3) (5 mg/kg) | 30.8±3 | OOR< | OOR < | 6.3±0.9 | 51.7±7 | 1223.3±78.9 | 172.0±19 | 1080.0±170 | 0.5±0.06 | 24.7±2.4 |
| VD3 (N=3) | 54.5±15 | OOR< | OOR < | 5.8±1.8 | 66.3±11 | 1526.7±211 | 144.7±14 | 2220.0±630 | 0.4±0.04 | 35.7±1.2 |
| ND3 (N=3) (0.025 mg/kg) | 37.0±8 | OOR < | OOR < | 12.1±5.17 | 52.3±3.53 | 1243.3±77.5 | 146.7±14.5 | 1386.7±402.9 | 0.4±0.03 | 33.3±1.2 |
| ND3 (N=3) (5 mg/kg) | 139.7±71 | OOR < | OOR < | 8.1±3.12 | 211.0±73 | 1076.7±167.4 | 297.3±87 | 1550.0±274 | 0.2±0.05 | 35.0±6.1 |
| CTRL (N=3) treated with physiological solution | 23.8±8 | OOR < | OOR < | 10.9±4.6 | 58.7±6.7 | 1520.0±38 | 132.7±17 | 1990.0±300 | 0.3±0.03 | 31.3±1.2 |

The data were not significantly different among groups as determined by one way analysis of variance.

RT-PCR experiments showed that the mRNA levels of *Pgc1-alpha* and *Cgrp* genes were significantly enhanced following i.v. **ND3** (5 mg/kg) treatment and after 14 days of follow-up in rat brain as determined by one way ANOVA (F = 4.11, p < 0.05), Bonferroni and Tukey HSD tests [Tukey HSD test *p* values: **ND3** (5 mg/kg) *vs* CTRL, p < 0.005]. The *Cgrp* gene was also up-regulated in the VD3 group [Tukey HSD test p values: VD3 *vs* CTRL, p < 0.005] (Figure 9). The expression level of *Atrogin-1, Murf-1, Bnip3, TrpV1, Caspase 3,* 8 and 9 genes was not significantly affected in brain (ANOVA one way, F =0.9). The *Tibialis Anterior* muscle genes of the same rats were not significantly affected by the i.v. treatments (ANOVA one way, F = 1.1) (Figure 9).

The effects of the highest doses of **ND3** (50 mg/kg) and **ND2** (50 mg/kg) formulations and their relative vehicles VD3 and VD2 were investigated in mice. **ND3** (50 mg/kg) and VD3 treatments induced a significant loss of body weight after a period of follow-up of 14 days *vs* all groups (ANOVA F = 5.22, p < 0.05) (Figure 10). This difference was determined by the **ND3** and VD3 groups as revealed by the Bonferroni and Tukey HSD tests **(ND3** *vs* CTRL, p < 0.05; **ND3** *vs* **ND2,** p < 0.020; **ND3** *vs* VD2, p < 0.005; VD3 *vs* CTRL p < 0.020; VD3 *vs* VD2, p < 0.05) (Figure 10).

No change of sampled wet organs weight among experimental groups (ANOVA one way, F = 1) was observed (Figure 11). The metabolic parameters were not significantly different among groups after 14 days of follow-up in mice (ANOVA one way, F = 0.94) (Table 5).

The i.v. **ND3** (50 mg/kg) and VD3 treatments significantly enhanced the *Lc3* and *Bnip3* genes expression of *Tibialis Anterior* muscle (F = 3.2, p < 0.05) as evaluated by the ANOVA one way analysis in mice (Figure 12). The *Lc3* gene was also significantly up-regulated following the **ND2** (50 mg/kg) (F = 2.2, p < 0.05). In contrast, the expression levels of *Atrogin-1, Murf-1, Pgcl alpha, UCP 1, Nfkb1, Caspase 3,* 9 and *Tnf alpha* genes were undetectable in *Tibialis Anterior* muscle after 14 days of follow-up even using pre-amplification methodology for total mRNA extraction suggesting a marked nucleotide down-regulation. Brain genes expression was not affected following treatments in mice (ANOVA one way, F = 0.92) (Figure 12).

The mRNA content was abnormally reduced in the muscle and brain of **ND3** (50 mg/kg) and VD3 treated mice *vs* controls (Table 7).

**Table** 7. mRNA content of *Tibialis Anterior* muscle and Brain following the i.v. administration of a single dose of F-DOPA formulations after a follow-up of 14 days in male C57BL/6J mice.

| Treatments | *Tibialis Anterioris* muscle mRNA (ng/mL) | Brain mRNA (ng/mL) |
|---|---|---|
| CTRL (N mice=4) | 221±12 | 7498±280 |
| ND3 (50 mg/kg) (N mice=4) | 46±7 * | 3916±100 * |
| VD3 (N mice=4) | 34±3 * | 4547+270 * |
| ND2 (50 mg/kg) (N mice=4) | 187±14 | 8055+460 |
| VD2 (N mice=4) | 195±10 | 5943+383 |

The experimental groups were: CTRL, controls mice treated with physiological solution; ND3, mice treated with acetic acid based F-DOPA (50 mg/kg); ND2, mice treated with lactate/$Na_2EDTA$ based F-DOPA (50 mg/kg); VD3, mice treated with the acetic acid vehicle solution; VD2, mice treated with lactate/$Na_2EDTA$ vehicle solution. The data represents the mean ± S.D. of mRNA content of *Tibialis Anterior* muscle and Brain. Data significantly different: * ND3 and VD3 vs control (CTRL) treated with physiological solution, (p<0.05) as determined by *student t test.*

Oxidative stress analysis in serum samples following a single dose i.v. administration of F-DOPA formulations and a follow-up of 14 days revealed no significant changes of the total antioxidant capacity of the serum samples from different animals expressed as Trolox equivalent in nmol/sample (μL) (Total Antiossidant Assay Kit, Sigma-Aldrich, MAK187) as well as of MDA levels used as an index of lipid peroxidation in mice (ANOVA one way, F = 0.81) [Lipid Peroxidation (MDA) Assay Kit, Sigma-Aldrich, MAK085].

## CONCLUSIONS

Based on the results of the preformulation studies reported here, the non-enzymatic degradation of F-DOPA in solution mainly occurs through oxidation at the catechol moiety. These findings definitely proved that the oxidation of F-DOPA in solution is markedly affected by pH and the kinetic parameters ($t_{90\%}$ values) follow a complex pH dependency and became fully base-dependent above pH 7.0. In addition, the stability of F-DOPA in aqueous solutions significantly increased by oxygen exclusion and due to the presence of a chelating agent. Hence, based on this body of evidence two new lactate-based formulations of F-DOPA, namely **ND1** and **ND2,** were prepared which proved to significantly

improve the chemical stability of F-DOPA in solution at pH 5.0, which is a pH value compatible with the direct infusion, with shelf lives greater than that reported for the commercially available preparation (IASOdopa®, above labeled as **ND3).** Furthermore, *in vitro* and *in vivo* investigations provided support to better cell tolerability of **ND2** formulation as compared to **ND1** and **ND3** formulations.

## EXPERIMENTAL SECTION

*Materials*

(2*S*)-2-Amino-3-(6-fluoro-3,4-dihydroxyphenyl)propanoic acid (6-fluoro-L-DOPA; F-DOPA), L-(+)-lactic acid, L-(+)-lactic acid sodium salt, EDTA disodium salt ($Na_2$EDTA) and all commercial reagent grade chemicals, including buffer components, were purchased from Sigma-Aldrich (Milan, Italy) and were used without further purification. The water was double-distilled and sterilized by autoclaving. All the aqueous solutions were deoxygenated and kept in tightly closed glass vials of type I under nitrogen until further use. All 5 mL vials and the rubbers stoppers, before their use, were first washed several times with distilled water and then dried by dry heat in an oven at 45 $\pm$ 2 °C in inverted position. All organic solvents were high performance liquid chromatography (HPLC) grade. All the pH measurements were made at 25 °C with a digital pH meter (SevenExcellence, Mettler Toledo).

*Preliminary formulation studies, pH-rate profile*

The loss of F-DOPA follows complex non first order kinetics, therefore shelf life values ($t_{90\%}$) were estimated from percentage initial remaining plots. To identify the optimum pH range at which formulate, $t_{90\%}$ values for the F-DOPA degradation at 25 °C were estimated in aqueous buffer solutions at various pH values ranging from 1 to 8. The ionic strength was adjusted to 0.15 M by the addition of NaCl. The following buffer systems were used: HCl, acetate (0.005, 0.02, 0.05 M), phosphate (0.005, 0.02, 0.05 M), to achieve pH values 1-3, 4-5.5, and 6.5-8, respectively. Additionally, possible buffer catalysis was investigated at pH = 4-8. At all the pH values, the degradation rate of F-DOPA was followed using the reversed-phase HPLC procedure reported below. Degradation of F-DOPA was initiated by the addition of an aliquot of F-DOPA stock solution (1 mg/mL) in deoxygenated sterilized double-distilled water to a 2 mL volumetric flask followed by the addition of a suitable buffered aqueous solution to produce an initial concentration of 0.1 M and the desired pH. Aliquots of 20 μL were withdrawn periodically from the stored sample and immediately injected into the HPLC. Quantification was performed by peak area analysis in relation to standard calibration curves. Shelf life values were calculated at various pH values ranging from 1 to 8 from the plots of peak area/peak area($t_0$) versus time and used to construct the pH-rate profile.

*HPLC analysis*

The HPLC system used to check the chemical stability of F-DOPA, related to its possible oxidation and racemization, consisted of a Waters Associates (Milford, MA, USA) model 1515 equipped with an isocratic pump, a Waters 2487 UV-vis detector, and the Breeze Software to analyze the chromatographic data. The HPLC analysis were performed on a reverse-phase column (Agilent Zorbax SB-Aq C8; 150 x 4.6 mm - 5 μm) kept at room temperature using detection wavelengths of 200 and 280 nm. The mobile phase consisted of 6.9 g/L solution of sodium dihydrogen phosphate adjusted to pH 2.4 with a 4.8 g/L solution of phosphoric acid, and the flow rate was 1 mL/min. The standard injection volume was 20 μL. Linear relationship between peak area and the concentration of the standard solutions was observed between 0.1 and 150 μM ($R^2$ > 0.999). To evaluate optical stability of F-DOPA, chromatograms were recorded on a teicoplanin aglycone-based chiral stationary phase (250 $\times$ 4.6 mm, particle size 5 μm, pore size 8 nm) CHIROBIOTIC (ASTEC, Whippany, NJ, USA), by eluting with a mixture 80% acetonitrile and 20% water, at a flow rate of 1 mL/min and a temperature of 25 °C.

*Physicochemical properties calculations*

p$K_a$ calculations were performed to determine the F-DOPA protonation states. ACD/Labs Suite p$K_a$ package release 9.0 (Advanced Chemistry Development, Inc., Toronto, Canada) was used for such calculations.

*in situ FT-IR experiments*

The degradation pathway and the progress of the F-DOPA oxidation in aqueous solutions were further investigated by in situ FT-IR experiments at pH 8.0 and 5.15 at 25 °C. The experiments were carried out using a Mettler-Toledo ReactIR 4000 and Ic15 instruments equipped with a diamond-tipped (DiComp®) probe (Spectra elaborated with

ConcIR® software), monitoring the C=O stretching of the F-DOPA and following new absorptions likely related to oxidized products (Castagnolo, D. et al. 2015; Mansueto, R. et al 2014). In a Schlenk tube equipped with a stirring bar and the React-IR probe, a 0.02 M F-DOPA aqueous solution at pH 5.15 was introduced *via* syringe at room temperature. Reference spectra for the pure solvent and F-DOPA were collected separately before starting the experiments. The solution was stirred for 10 min, to verify the stability of the readout, and spectra collected every 30 sec. The solution was then stirred for 24 h and constantly monitored by IR spectroscopy.

*Preparation of F-DOPA formulations*

Based on the results obtained in the preliminary formulation studies, two different formulations of F-DOPA, namely **ND1** and **ND2,** were prepared and subjected to quality control and stability studies. In particular, exactly weighted amounts of F-DOPA were solubilized in 1 mL of 1 mM lactate buffer aqueous solution (pH 5.0) in the presence of 1 mM $Na_2EDTA$ with **(ND1)** or without **(ND2)** ionic strength adjusted to 150 mM by adding NaCl. The highest tested concentration of F-DOPA was of 10 mg/mL. After preparation, the samples were kept in tightly closed glass vials of type I under nitrogen in the dark until further analysis. F-DOPA delivered in an aqueous solution of acetic acid (1.05 mg/mL) was used as control in the *in vitro* experiments, namely formulation **ND3,** resembling the commercially available IASOdopa®.

*Chemical stability studies of F-DOPA in the parenteral formulations*

The stability of a 100 μM solution of F-DOPA in the parenteral formulations **ND1** and **ND2** was measured at 25 and 40 °C. F-DOPA was assayed just after the preparation of the batch at determined intervals of time. The F-DOPA degradation rate was investigated by HPLC as described above. The pH value was measured during the experiments and the solutions were also visually inspected for color changing and precipitate appearance.

*Cyclic temperature stress testing*

F-DOPA parenteral formulations freshly prepared were sealed in glass vials under an inert atmosphere and stored in the dark for a total of 60 h, 12 h for each step, in a climatic chamber (Climacell, MMM Group) at temperature and relative humidity (RH) values set as follow: 4 ± 2 °C, 25 ± 2 °C/60 ± 5% RH, 40 ± 2 °C/60 ± 5% RH, 25 ± 2 °C/60 ± 5% RH, and 4 ± 2 °C. At the end of the cyclic temperature stress test, the sealed vials of the formulations were visually inspected against black and white backgrounds to see the occurring changes, such as color, turbidity and precipitate formation during the storage period. F-DOPA content and purity were determined by HPLC analysis following the procedure described above. The percent residual drug for each formulation at the end of the incubation period was calculated considering the initial drug content for each formulation to be 100%.

*Cell lines and culture.*

The human neuroblastoma SH-SY5Y and embryonal kidney tsA201 cell lines were purchased by Sigma Aldrich (ECACC) (SIGMA Chemical Co., Milan, Italy). The cells were cultured in DMEM+ composed by Dulbecco's Modified Eagle's Medium (DMEM) supplemented with 10% Fetal Bovine Serum (FBS), 1% antibiotics (penicilline and streptomycine) and 1% L-glutamine in a humidified atmosphere containing 5% $CO_2$. All culture medium components were from EuroClone (EuroClone S.p.A., Milan, Italy).

Single fibers were isolated from the *Flexor Digitorum Brevis* (FDB) muscles dissected from male C57BL6 mice (N° of mice = 3). Isolated fibers were obtained by incubating the FDB muscles with Dulbecco's modified Eagle's medium (DMEM+) solution composed by IX antibiotics (1%), L-glutamine (1%), FBS (10%) (EuroClone, S.p.A. Milan, Italy) enriched with 0.3 mg/ml collagenase type XI-S (Sigma Chemical Co.).

For *"in vitro"* cell viability tests, the F-DOPA powder was directly solubilized in the complete cell culture medium (DMEM+). Stock solutions of **ND1, ND2** and **ND3** formulations were prepared at the concentration of 10 mg/ml. Microliter amounts of the stock solutions were then added to DMEM as needed, to obtain the final concentrations.

*"In vitro " cell viability experiments.*

The fibers were plated, counted under a microscope, and incubated for 3h with increasing concentrations of F-DOPA, **ND1, ND2** and **ND3** formulations and an imaging analysis was then performed. At the end of the incubation time, the cytotoxicity expressed as mortality rate with respect to the control condition was evaluated by counting the fibers before and after the incubation period.

Cell viability tests were performed on human kidney tsA201 cell line and human neuroblastoma SH-SY5Y cell line (Sigma-Aldrich, Milan, Italy) by evaluating cell morphology changes using Scepter™ Sensor technology. The Scepter™2.0 cell counter (MERK-Millipore, USA) is compatible with 60 and 40 μm sensors; in our experiments the 60 μm sensor for particles between 6 and 36 μm was used. In these experiments, the cells were counted, plated, and incubated for 3 h in the absence or in the presence **of ND1, ND2** and **ND3** formulations at the concentrations of 0.005 mg/ml, 0.05 mg/ml, 0.2 mg/ml and 0.5 mg/ml.

*"In vivo" experiments: animal groups and dosing.*

Male Wistar rats weighing 400 ± 33 g (Charles River Lab., Calco, Italy) (N° of total rats = 48) and male C57BL/6J mice weighing 44 ± 4 g (Charles River Lab., Calco, Italy) (N° of total mice = 19) were used. The animals were divided in different experimental groups: Control untreated CTRLU, control CTRL, **ND2** 0.025 mg/kg, **ND2** 0.1 mg/kg, **ND2** 5 mg/kg in rat and 50 mg/kg in mice, and the ND2-vehicle (VD2); **ND3** 0.025 mg/kg, **ND3** 0.1 mg/kg, **ND3** 5 mg/kg in rat and 50 mg/kg in mice, and the ND3-vehicle (VD3). Stock solutions of the formulations were prepared at the concentration of 10 mg/ml. Different concentrations of the **ND2** and **ND3** formulations were obtained by addition of physiological solution immediately before the administration to avoid fast F-DOPA degradation. For each group, the solutions were injected as a bolus of maximal volume of 0.2 ml which was adjusted on the basis of the body weight over a time period of 30 seconds using a sterile plastic syringe equipped with a 27G needle for i.m., s.c., i.p. and i.v. administrations.

The control untreated CTRL U did not receive any treatment and was not sacrificed at the end of the protocols. The CTRL group received sterile physiological solution.

The **ND2** and **ND3** 0.025 mg/kg groups received 0.01 mg mass dose of F-DOPA, which is in the range of the human therapeutic dose of 1.75 mg for a man of 70 kg of body weight normalized to the rat body weight of 400 g and injected at the concentration (0.05 mg/ml x injected volume).

The **ND2** and **ND3** 0.1 mg/kg groups received 0.04 mg mass dose of F-DOPA at the concentration (0.2 mg/ml concentration x injected volume).

The **ND2** and ND3 5 mg/kg in rat and **ND2** and **ND3** 50 mg/kg in mice groups received 2 mg mass dose (10 mg/ml x injected volume) of F-DOPA which is the highest human therapeutic dose corresponding to an activity of 280 MBq, assuming a specific radioactivity of [18F]-F-DOPA of 30 GBq / mmol (IASOdopa®, SPC 2016).

The vehicle groups were rats and mice treated with equal amounts of the formulations free of F-DOPA.

*Ethics statement.*

The animal care and treatments were performed according to the Italian D.L. 4 march 2014, n. 26, and 2010/63/EU law on Animal Protection Used for Scientific Experiments. The protocols design was based on the Replacement, Reduction and Refinement principles. In *Single Dose Extended Toxicity* i.v. (SDET) study, a statistically significant evaluation of the endpoint was reached using a reduced number of animals and the sample size was calculated assuming a power of 0.8-0.9. In local tolerability experiments, in the absence of clinical reactions, the number of rats was kept at the minimum by using the same rats for different protocols. The protocols were approved at March 2013 by the Italian Health Department (Art. 9 del Decreto Legislativo 116/92, Decreto no. 33/2000-B del Dipartimento degli Alimenti e Nutrizione e della Sanità Pubblica) and performed under the supervision of a local veterinary official. The local tolerability experiments were performed in conscious rats and caused a moderate pain in the treated animals. The *Single Dose Extended Toxicity* i.v. study in rats and mice produced a mild-moderate pain in the treated animals. The i.v. treatments were performed under a mild anesthesia obtained using an oral gavage administration of chloral hydrate 200 mg/ml (1.14 g/kg body weight). The blood sampling was performed under deep anesthesia produced by i.p. injection of chloral hydrate (2 g/kg body weight). The animals were sacrificed by cervical dislocation. The organs collection was performed in death animals. In agreement with the 2010/63/EU law no positive controls were used in the present work.

*Local tolerability tests in rats.*

These experiments were performed in conscious rats (N° of rats = 24). The i.m. tolerability experiments consisted in the administration of the formulations and relative vehicles into the *Tibialis Anterior* muscle area. The rats were shaved on their legs and divided in different experimental groups: controls CTRL (N° of rats = 3), **ND2** (0.1 mg/kg) (N° of rats = 3), **ND2** (5 mg/kg) (N° of rats = 3), ND3 (0.1 mg/kg) (N° of rats = 3) and **ND3** (5 mg/kg) (N° of rats = 3). For each group, a volume of 0.2 ml of the drug solutions was injected into the left leg of the rat and the corresponding vehicle on the right leg. The control group received 0.2 ml of the physiological solution on the left and no treatment on right legs. The rats were monitored for skin local reactions such as redness, darkening and oedema formation within 24h following injection. The rats showing signs of adverse reactions following i.m. injections were scarified and replaced by new rats to enter the new experimental protocol. The rats enter the new protocols after 30 days of washout. The same experimental design, animal groups and dosing was used for the evaluation of the animal reaction to hindpaw injection (h.i.) of the formulations. The rats were monitored for hindlimb licking, shaking and withdrawal or oedema formation within 24h

following the h.i. injection.

i.m. and h.i. protocols:

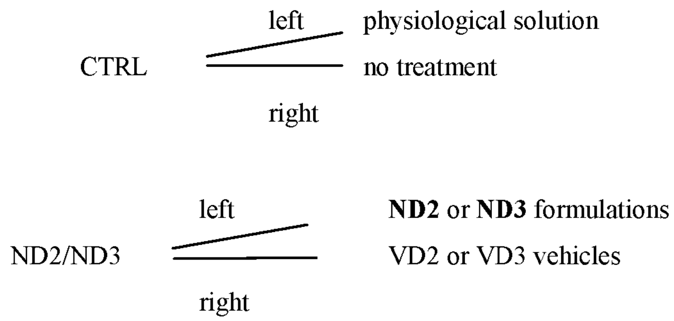


In the absence of adverse reactions, the same animals were used for s.c. and i.p. tolerability tests after 30 days of washout period. The animal groups and dosing were: controls CTRL U (N° of rats = 3), ND2 (5 mg/kg) (N° of rats = 3), VD2 (N° of rats = 3); controls CTRL (N° of rats = 3), ND3 (5 mg/kg) (N° of rats = 3) and VD3 (N° of rats = 3).

These experiments were performed observing the animal adverse reactions to i.p. and s.c. administrations in locally shaved rats of the highest doses of the same formulations and vehicles in terms of convulsion, piloerections or oedema within 24h from the administration.

*s.c. and i.p. protocols:*

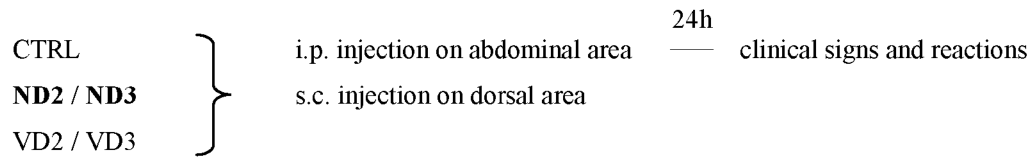


In the presence of reactions, the rats were rapidly sacrificed to collect muscles and organs. The organs were collected in the formaldehyde solution (10%) for histopathological analysis and stored at -80°C for gene expression analysis. Cardiac blood and serum samples were conserved at -20 C° using appropriate anticoagulants.

*Single Dose Extended Toxicity Study in rats and mice.*

According to the international guidelines for radiotracers development, the F-DOPA formulations under investigation are *"not first time in men"* and a *Single Dose Extended Toxicity* study of intravenous administration of the formulations can be performed (EMA/CHMP/CVMP/JEG-3Rs/169839/2011-Rev.1).

The aims of this experiment were the evaluation of acute toxicity of a single human and animal dose of drugs and evaluation of reversibility of the observed effects after 14 days of follow-up in rat (N° of rats = 24) and mice (N° of mice = 16).

*Primary endpoints:* body weight changes.

*Secondary endpoints:* clinical signs, organs weight changes, biochemical parameters, gene expression of inflammatory and cell death markers, plasmatic cytokines, oxidative stress.

| i.v. protocol | 0 day | 1 day | 13 days | 14 days |
|---|---|---|---|---|
| | ↓ | ↓ | ↓ | ↓ |
| Experimental conditions | Single dose administration | Follow-up | Metabolic cage | Animal sacrifice |

The body weight and organs weight were evaluated in 24h fasted animals by using digital analytical balances AV114C 500 g and 100 g, respectively, and data were store for further analysis (OHAUS corp. NJ, U.S.A.). The animals groups were: controls CTRL U (N° of rats = 3, N° of mice = 4), **ND2** (0.025 mg/kg) (N° of rats = 3), **ND2** (0.1 mg/kg) (N° of rats = 3), **ND2** (5 mg/kg) (N° of rats = 3), **ND2** (50 mg/kg) (N° of mice = 4), controls CTRL (N° of rats = 3, N° of mice = 4), **ND3** (0.025 mg/kg) (N° of rats = 3), **ND3** (0.1 mg/kg) (N° of rats = 3) and **ND3** (5 mg/kg) (N° of rats = 3), **ND3** (50 mg/kg) (N° of mice = 4).

Each animal was weighed before treatment and anesthetized with oral administration of chloral hydrate (1.14 g / kg body weight), and then intravenous administration of the formulation under investigations in the tail vein of the animal was performed in unconscious animals. After administration, the animals were placed in individual metabolic cages for not more than 2 days to reduce discomfort and distress and monitored for the following parameters: urine and feces production, consumption of water and food after 14 days of follow-up. At the end of the follow-up, a deep anesthesia was produced by a single i.p. injection of chloral hydrate (2 g/kg body weight) and the intracardiac blood samples were collected from unconscious living animals. Serum samples were conserved at -20 C° using appropriate anticoagulants for the evaluation of plasma biomarkers of toxicity.

*Tibialis Anterior* (TA), *Extensor Digitorum Longus* (EDL) and *Soleus* (SOL) muscles, right and left kidney, heart, liver, lung and brain were removed from the animals sacrificed by vertical dislocation under deep anesthesia, were wiped-off of liquid from the surface, weighted and stored at -80° C for gene expression analysis.

*Biochemical analysis of plasma markers.*

The cytokines serum levels such as IL-1b, IL-6 and TNF alpha as indices of inflammation were evaluated using a Fluorescence based Bioplex-Luminex multitargets technology (BIOCLARMA Srl, via M. Cristina, 121 - 10126 Torino - Italy). The serum cardiac troponin C, the aminotransferases (ALT and AST), the amylase, the creatine kinase (CK), the creatinine and urea used as indices of heart, liver, pancreatic, skeletal muscle and renal damages, respectively, were investigated by colorimetric and ELISA methodologies.

*Isolation of total RNA, reverse transcription and real-time PCR.*

All tissues for gene expression analysis were snap frozen in liquid nitrogen soon after removal and stored at -80°C until use. For tibialis muscles and brain, the tissues were pulverized with liquid nitrogen before RNA extraction and the total RNA was isolated with Trizol reagent (Invitrogen Life Technologies). For each tibialis muscles of mice, total RNA was isolated by an RNeasy Fibrous Tissue Mini Kit (Qiagen C.N. 74704, Valencia) and quantified using a spectrophotometer (ND-1000 NanoDrop, Thermo Scientific, USA). To perform reverse transcription, 400 ng of total RNA were added to 1 mL dNTP mix 10 mM (Roche N.C. 11277049001, Switzerland) and 1 mL Random Hexamers 50 mM (Life Technologies C.N. n808-0127, USA) and incubated at 65°C for 5 min. Afterward, 4 mL 5X First Standard Buffer (Life Technologies C.N. Y02321), 2 mL 0.1 mM DTT (Life Technologies C.N. Y00147) and 1 mL Recombinant RNasin Ribonuclease Inhibitor 40 U/mL (Promega, C.N. N2511, USA) were added and incubated at 42°C for 2 min. One microliter of Super Script II Reverse Trascriptase 200 U/mL (Life Technologies C.N. 18064-014) was added to each solution and incubated at 25°C for 10 min, at 42°C for 50 min and at 70°C for 15 min. Real-time PCR was performed in duplicated using the Applied Biosystems Real-time PCR 7500 Fast system (USA), MicroAmp Fast Optical 96-Well Reaction Plate 0.1 mL Life Technologies C.N. 4346906) and MicroAmp Optical Adhesive Film (Life Technologies C.N. 4311971). Each reaction was carried in duplicated on a single plex reaction. The setup of reactions consisted of 8 ng cDNA, 0.5 mL of TaqManGeneExpression Assays (Life Technologies), 5 mL of TaqMan Universal PCR master mix No AmpErase UNG (2x) (Life Technologies C.N. 4324018) and nuclease-free water not diethylpyrocarbonate (DEPC-Treated)

(Life Technologies C.N. AM9930) for a final volume of 10 mL. The following RT-TaqMan-PCR conditions were as follows: step 1: 95°C for 20s, step 2: 95°C for 3s and step 3: 60°C for 30s; steps 2 and 3 were repeated 40 times. The results were compared with a relative standard curve obtained by five points of 1:4 serial dilutions. The mRNA expression of the genes was normalized to the best housekeeping genes glyceraldehyde-3-phosphate dehydrogenase *(Gapdh)* selected from beta-actin (*β-Actin*) and beta-2 microglobulin (*β-2m*) and *Gapdh.* TaqMan Hydrolysis primer and probe gene expression assays were ordered with the assay IDs reported in Table 2, by Life Technologies except for *β-Actin* of mice, where the assay was made with the following sequences: forward primer: 5'-CCAGATCATGTTTGAGACCTTCAA-3' (SEQ ID No.: 1), reverse primer: 5'-CATACAGGGACAGCACAGCCTY-3' (SEQ ID No.: 2) and Probe: VD3C-ACCCCAGCCATGTACGTA-MGB (SEQ ID No.: 3). For genes that are little expressed, a pre-amplification by TaqMan PreAmp Master Mix (Life Technologies C.N. 4391128) was made before the real-time experiments. The set up of pre-amplification consisted by 250 ng of reverse-transcribed (in 12.5 mL volume), 25 mL of TaqMan PreAmp Master Mix (2x) and 12.5 mL of Pool Assay 0.2x. The Pool Assay for rats and mice were reported in Table 8. The solution was incubated at 50°C for 2 min, 95°C for 10 min. and for 40 cycles of 95°C for 15s and 60°C for 1 min. The RT-PCR experiments and analysis were performed in agreement with the MIQE guidelines for qPCR). **Table 8.** RT-PCR gene primers.

| Gene | Protein | Id assay code |
|---|---|---|
| Rat | | |
| *Fbxo32* | Atroginl | Rn_00591730_ml |
| *Casp3* | Caspasi3 | Rn_0563902_ml |
| *Casp9* | Caspasi9 | Rn_00581212_ml |
| *Trim63* | Murf_1 | Rn_00590197_ml |
| *Map3k8* | Mitogen activated protein kinase kinase kinase 8 | Rn_01538561_ml |
| *Bnip3* | Bcl2/adeno VD3 rus elb 19kda interacting protein | Rn_00821446_g1 |
| *Tnfa* | Tumor necrosis factor alpha | Rn_99999017_ml |
| *Map1lc3a* | Lc3 | Rn_01536227_ml |
| *Pargc1a* | Pgclalpha | Rn_00580241_ml |
| *NfKb1* | Nuclear factor of kappa light polypeptide gene enhancer in b cells | Rn_01399583_ml |
| *Casp8* | Caspasi8 | Rn_00574069_ml |
| *Trpv1* | Transient receptor potential cation channel, subfamily v, member 1 | Rn_00583117_ml |
| *Cgrp* | Calcitonin gene related peptide | Rn01511353_g1 |
| Mice | | |
| *Bnip3* | Bcl2/adenoVD3rus elb 19kda interacting protein | Mm_01275600_g1 |
| *Map1lc3a* | Lc3 | Mm_00458724_ml |
| *Pargc1a* | Pgclalpha | Mm_01208853_m1 |
| *Ucp1* | Uncoupling protein 1 | Mm_01244861_m1 |
| *Trim63* | Murf_1 | Mm_01185221_m1 |
| *Fbxo32* | Atroginl | Mm_00499523_m1 |
| *NjKb1* | Nuclear factor of kappa light polypeptide gene enhancer in b cells | Mm_00476361_m1 |
| *Tnfa* | Tumor necrosis factor alpha | Mm_99999068_m1 |
| *Casp3* | Caspasi3 | Mm01195085_m1 |
| *Casp9* | Caspasi9 | Mm00516563_m1 |

*Histological analysis of rat Tibialis Anterior muscle section.*

Fixed muscle tissue specimens were washed in water, dehydrated in ethanol, cleared in xylene, embedded in paraffin, and cut into serial sections of 10 μm of thickness from muscles stained with haematoxylin and eosin to determine the number of nuclei appearing as blue colored and cytosolic compartment red colored. The absolute values of both nuclei and capillaries of each fiber are relative values obtained by dividing them by the cross sectional area (CSA) expressed in · $m^2$ of the corresponding fiber. This staining did not distinguish myonuclei from other nucleus types (intra fibers nuclei, and nuclei of the satellite cells and capillaries). The number of nuclei was obtained by counting all the nuclei around each individual fiber. Images from 10 random fields were acquired for the ten stained sections of each specimen using a D 4000 Leica DMLS microscope equipped with a camera and image analyzer (NIS elements-BR-Nikon). The cross-sectional area of muscle fibers was measured with QWin software (Leica).

*Oxidative stress in mice.*

The total antioxidant capacity of the serum samples from different mice was evaluated and expressed as Trolox equivalent in nmol /sample (μL) (Total Antiossidant Assay Kit, Sigma-Aldrich, MAK187). The malondialdehyde levels were evaluated in the serum sample of mice [Lipid Peroxidation (MDA) Assay Kit, Sigma-Aldrich, MAK085].

*Data analysis and statistics.*

The data are expressed as mean ± S.E.M..

The mortality rate of the fibers and cells was calculated by the following equation:

$$[(N_{treated}/N_{CTRL})x100]-100$$

where $N_{treated}$ is the number of the treated cells and $N_{CTRL}$ is the number of untreated cells.

In *"in vivo"* experiments, the changes of muscles and organs wet weight normalized to body weight was calculated as follow:

organ weight (g) / body weight (g) after a follow-up of 14 days in rat
organ weight (g) / body weight (g) x 1000 after a follow-up of 14 days in mice

The percent change of body weight was calculated as follows:

$$\text{percentage change of body weight (\%)} = [\text{body weight (g) } t_{14} / \text{body weight (g) } t_0 \text{ x } 100]-100$$

where the body weight $t_0$ is the weight before the administration at time and the body weight $t_{14}$ is the weight after a follow-up of 14 days.

The following three parametric logistic function was used to fit the concentrationmortality data of different F-DOPA formulations:

$$\text{percentage change of the mortality rate (\%)} = E_{max} (1 + EC_{50}/DOSE)^{slope}$$

where $E_{max}$, is the maximal drug effect; $EC_{50}$, is the concentration needed to cause the 50% percent change of the maximal effect; DOSE, is the applied concentration; slope, is the hill slope of the curve (SIGMAPlot 10.0, Systat Software, Inc.).

Data were exported and further analysed using Excel software (Microsoft Office 2010). Differences within and between drug treatment groups were evaluated using one way ANOVA, Bonferroni and Tukey HSD tests correction to counteract for multiple comparison and test for the significance of each individual hypothesis at a significant level of $\alpha = 0.05$.

Student t-test, at $p < 0.05$ and $p < 0.001$ levels of significance, was used to test for significance between means.

In *Single Dose Extended Toxicity* study, a sample size of 24 animals (N° = 3 rat/group) for 8 data groups based on the one way ANOVA analysis was calculated assuming a maximal 30% changes of the body weight reduction following treatment with the F-DOPA formulations, the same standard deviation, an error $\alpha = 0.05$ and a power of the study of 0.8. In mice experiments, a sample size 16 animals (N° = 4 mice/group) for 4 data groups was calculated assuming a maximal 30% changes of the body weight reduction following treatment with the F-DOPA formulations, the same standard deviation, an error $\alpha = 0.05$ and a power of the study of 0.89 (G*Power software 3.1.9.2, IDRE, UCLA U.S.A.).

## REFERENCES

1) Dopacis ® (2013). Summary of Product Characteristic. Available at: http://www.ibamolecular.eu/sites/default/files/DOPACIS%20SPC 0.pdf
2) EMA/CHMP/CVMP/JEG-3Rs/169839/2011-Rev.1.
3) IASOdopa® (2016) Summary of Product Characteristic. Available at: http://www.iason.eu/fileadmin/user upload/SPC/IASOdopa_english.pdf
4) Package leaflet: information for the patient. IASOdopa 0.3 GBq/mL, concentrate for solution for injection. 6-fluoro-($^{18}$F)-L-dihydroxyphenylalanine (or 6-fluoro-($^{18}$F)-L-dopa). Country: France, date of registration November 11, 2006. Available at: http://www.iason.eu/fileadmin/user_upload/PIL/IASOdopa_pil_english.pdf

## Claims

1. Pharmaceutical formulation comprising 3,4-dihydroxy-6-[18F]-fluoro-L-phenylalanine ([$^{18}$F]F-DOPA) and at least one buffering agent in a aqueous vehicle, wherein the formulation has a pH value about 5, wherein the formulation comprises at least one metal cation chelating agent, wherein the at least one buffering agent is lactate, and wherein the formulation is compatible with direct infusion.

2. Pharmaceutical formulation according to claim 1, wherein the formulation comprises at least one of: at least one ionic force regulator agent, at least one preservative agent, at least one isotonic agent.

3. Pharmaceutical formulation according to claim 1, wherein the at least one metal cation chelating agent is selected from: EDTA, citric acid, tartaric acid, and salts thereof.

4. Pharmaceutical formulation according to any one of claims 2 to 3, wherein the at least one preservative agent is selected from sulfurated compounds, preferably sodium bisulfite and sodium sulfite, ascorbic acid and boric acid and salts thereof.

5. Pharmaceutical formulation according to any one of claims 2 to 4, wherein the at least one ionic force regulator agent is sodium chloride.

6. Pharmaceutical formulation according to any one of claims 2 to 5, wherein the at least one isotonic agent is selected from sodium chloride, glucose.

7. Pharmaceutical formulation according to any one of claims 1 to 6, wherein the aqueous vehicle is substantially free of oxygen species, preferably the aqueous vehicle is deoxygenated water for pharmaceutical use, more preferably is deoxygenated double distilled water.

8. Pharmaceutical formulation according to any one of claims 1 to 7 for use as radiotracer in an imaging diagnostic technique, preferably a positron emission tomography (PET).

9. Pharmaceutical formulation according to any one of claims 1 to 7 for use in diagnosis of neurodegenerative diseases, preferably Parkinson’s disease.

10. Pharmaceutical formulation according to any one of claims 1 to 7 for use in diagnosis of tumors, preferably neuroendocrine tumors (NETs).

11. Pharmaceutical formulation according to any one of claims 1 to 7 for use in diagnosis of a tumor in a subject, wherein the tumor is preferably selected from: brain tumors, pancreas tumor, medullary thyroid cancer, catecholamine-producing tumors, carcinoid tumors of midgut, ovarian cancer, sentinel lymph nodes.

**Patentansprüche**

1. Pharmazeutische Formulierung, umfassend 3,4-Dihydroxy-6-[18F]-fluor-L-phenylalanin ([$^{18}$F]F-DOPA) und mindestens ein Puffermittel in einer wässrigen Trägersubstanz, wobei die Formulierung einen pH-Wert von etwa 5 aufweist, wobei die Formulierung mindestens einen Metallkation-Chelatbildner umfasst, wobei das mindestens eine Puffermittel ein Laktat ist, und wobei die Formulierung mit einer direkten Infusion kompatibel ist.

2. Pharmazeutische Formulierung nach Anspruch 1, wobei die Formulierung mindestens eines der folgenden umfasst: mindestens ein ionisches Kraftreglermittel, mindestens ein Konservierungsmittel, mindestens ein isotonisches Mittel.

3. Pharmazeutische Formulierung nach Anspruch 1, wobei der mindestens eine Metallkation-Chelatbildner aus folgenden ausgewählt ist: EDTA, Citronensäure, Weinsäure und Salze davon.

4. Pharmazeutische Formulierung nach einem der Ansprüche 2 bis 3, wobei das mindestens eine Konservierungsmittel aus schwefelhaltigen Verbindungen ausgewählt ist, vorzugsweise Natriumbisulfit und Natriumsulfit, Ascorbinsäure und Borsäure sowie Salze davon.

5. Pharmazeutische Formulierung nach einem der Ansprüche 2 bis 4, wobei das mindestens eine ionische Kraftreglermittel Natriumchlorid ist.

6. Pharmazeutische Formulierung nach einem der Ansprüche 2 bis 5, wobei das mindestens eine isotonische Mittel aus Natriumchlorid und Glucose ausgewählt ist.

7. Pharmazeutische Formulierung nach einem der Ansprüche 1 bis 6, wobei die wässrige Trägersubstanz im Wesentlichen frei von Sauerstoffspezies ist; vorzugsweise ist die wässrige Trägersubstanz sauerstoffarmes Wasser für pharmazeutische Zwecke, insbesondere ist sie sauerstoffarmes, bidestilliertes Wasser.

**8.** Pharmazeutische Formulierung nach einem der Ansprüche 1 bis 7 zur Verwendung als Radiotracer bei einem diagnostischen Bildgebungsverfahren, vorzugsweise einer Positronen-Emissions-Tomographie (PET).

**9.** Pharmazeutische Formulierung nach einem der Ansprüche 1 bis 7 zur Verwendung bei der Diagnose von neurodegenerativen Erkrankungen, vorzugsweise Morbus Parkinson.

**10.** Pharmazeutische Formulierung nach einem der Ansprüche 1 bis 7 zur Verwendung bei der Diagnose von Tumoren, vorzugsweise neuroendokrine Tumore (NET).

**11.** Pharmazeutische Formulierung nach einem der Ansprüche 1 bis 7 zur Verwendung bei der Diagnose eines Tumors bei einem Individuum, wobei der Tumor vorzugsweise aus folgenden ausgewählt ist: Hirntumore, Pankreastumor, medulläres Schilddrüsenkarzinom, Katecholaminproduzierende Tumore, karzinoide Tumore des Mitteldarms, Eierstockkrebs, Sentinel-Lymphknoten.

**Revendications**

**1.** Formulation pharmaceutique comprenant de la 3,4-dihydroxy-6-[18F]-fluoro-L-phénylalanine ([$^{18}$F]F-DOPA) et au moins un agent tampon dans un véhicule aqueux, où la formulation a un pH d'environ 5, où la formulation comprend au moins un agent chélatant les cations métalliques, où le au moins un agent tampon est un lactate, et où la formulation est compatible avec une administration directe.

**2.** Formulation pharmaceutique selon la revendication 1, où la formulation comprend au moins l'un de: au moins un agent régulateur de force ionique, au moins un agent conservateur, au moins un agent isotonique.

**3.** Formulation pharmaceutique selon la revendication 1, où le au moins un agent chélatant les cations métalliques est choisi parmi: l'EDTA, l'acide citrique, l'acide tartrique et leurs sels.

**4.** Formulation pharmaceutique selon l'une quelconque des revendications 2 à 3, où le au moins un agent conservateur est choisi parmi les composés soufrés, de préférence le bisulfite de sodium et le sulfite de sodium, l'acide ascorbique et l'acide borique et leurs sels.

**5.** Formulation pharmaceutique selon l'une quelconque des revendications 2 à 4, où le au moins un agent régulateur de force ionique est le chlorure de sodium.

**6.** Formulation pharmaceutique selon l'une quelconque des revendications 2 à 5, où le au moins un agent isotonique est choisi parmi le chlorure de sodium, le glucose.

**7.** Formulation pharmaceutique selon l'une quelconque des revendications 1 à 6, où le véhicule aqueux est sensiblement exempt d'espèces d'oxygène, de préférence le véhicule aqueux est l'eau désoxygénée à usage pharmaceutique, de préférence encore est l'eau bidistillée désoxygénée.

**8.** Formulation pharmaceutique selon l'une quelconque des revendications 1 à 7 destinée à être utilisée comme radiotraceur dans une technique d'imagerie diagnostique, de préférence une tomographie par émission de positrons (TEP).

**9.** Formulation pharmaceutique selon l'une quelconque des revendications 1 à 7 destinée à être utilisée dans le diagnostic des maladies neurodégénératives, de préférence la maladie de Parkinson.

**10.** Formulation pharmaceutique selon l'une quelconque des revendications 1 à 7 destinée à être utilisée dans le diagnostic des tumeurs, de préférence des tumeurs neuroendocrines (TNE).

**11.** Formulation pharmaceutique selon l'une quelconque des revendications 1 à 7 destinée à être utilisée dans le diagnostic d'une tumeur chez un sujet, où la tumeur est choisie de préférence parmi: les tumeurs cérébrales, les tumeurs du pancréas, le cancer médullaire de la thyroïde, les tumeurs produisant des catécholamines, les tumeurs carcinoïdes de l'intestin moyen, le cancer de l'ovaire, les ganglions lymphatiques sentinelles.

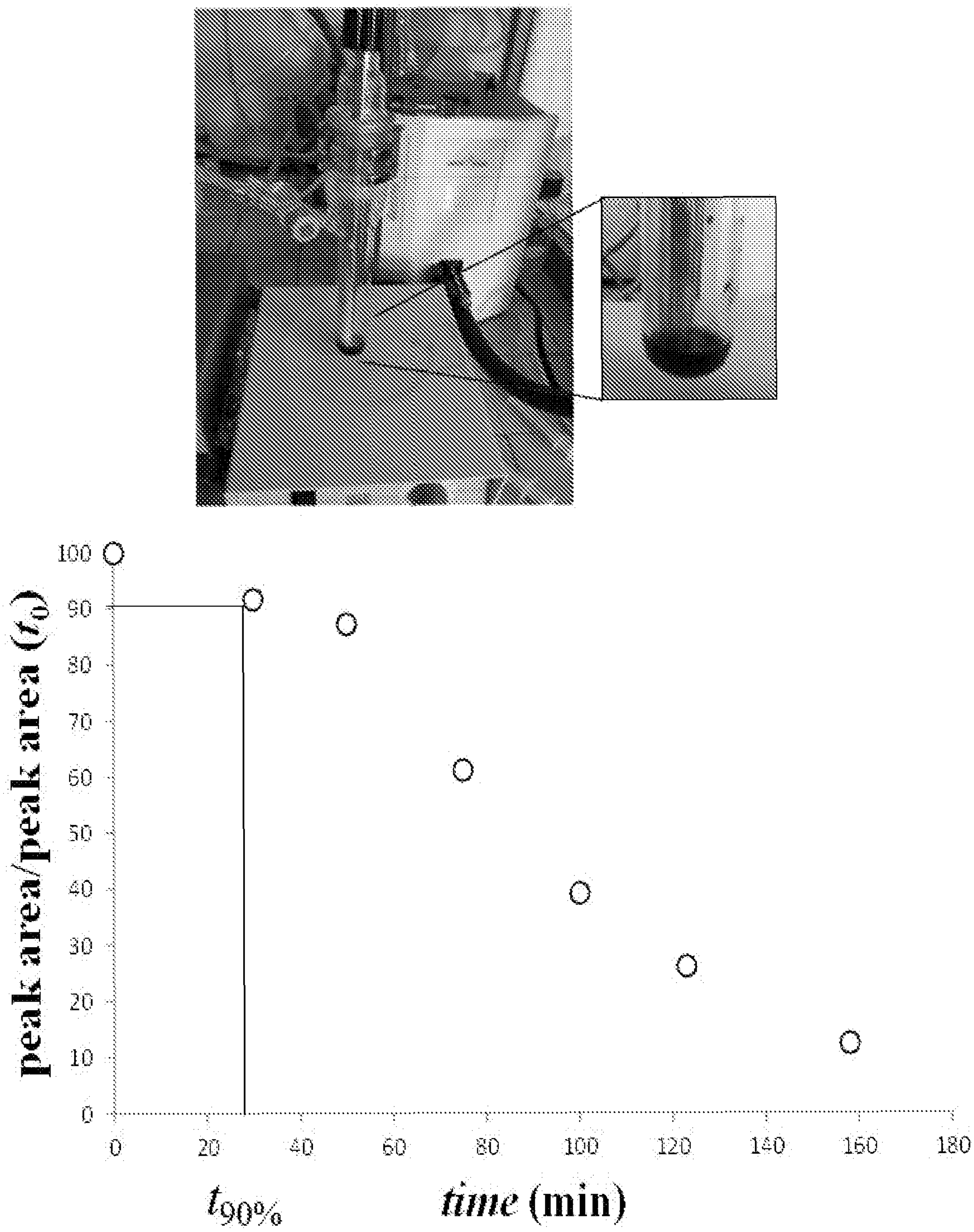
100
90
80
70
60
50
40
30
20
10
0
0
20
40
60
80
100
120
140
160
180
peak area/peak area ($t_0$)
$t_{90\%}$
*time* (min)

Figure 1

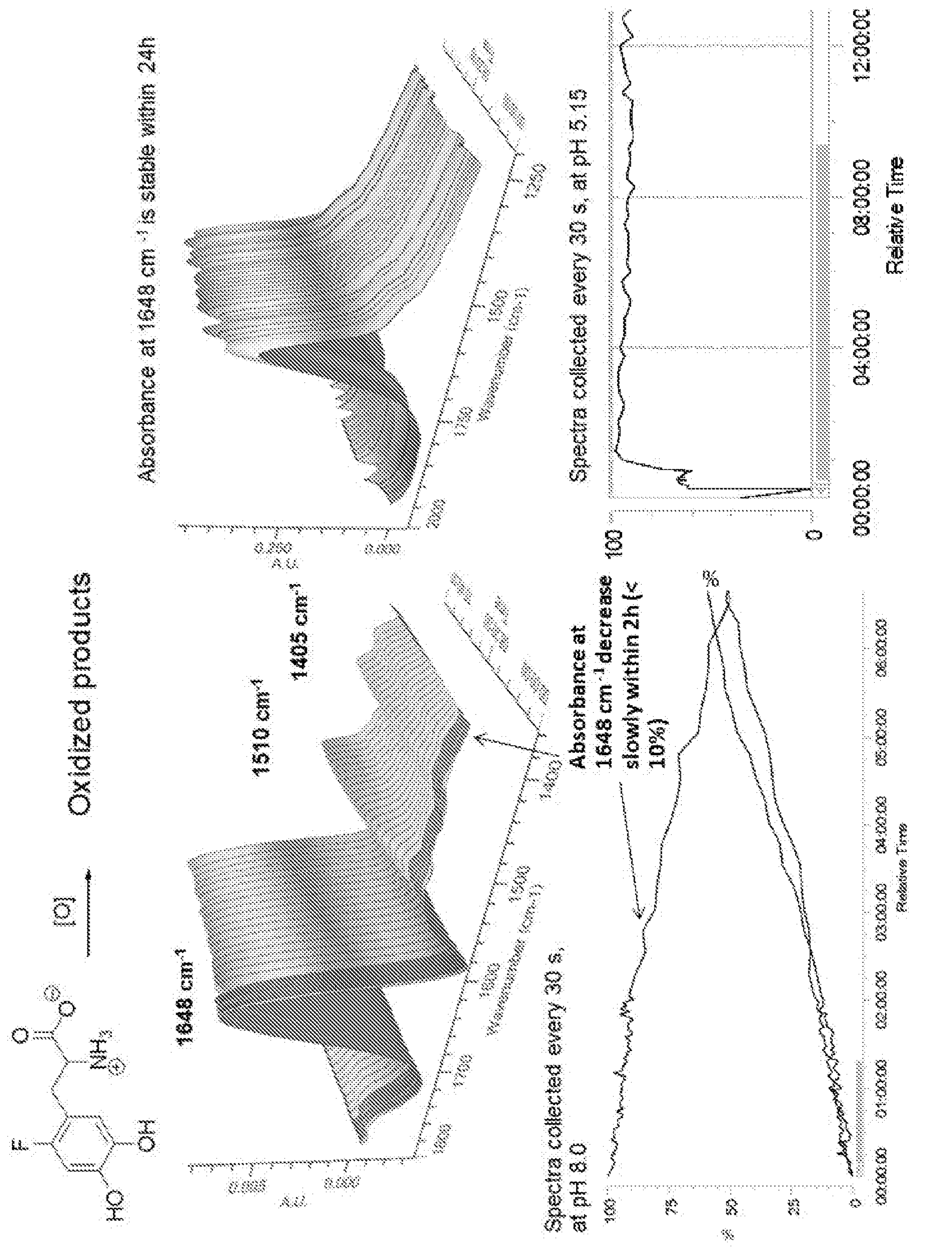

Oxidized products
[O]
1648 cm-1
1510 cm-1
1405 cm-1
Absorbance at 1648 cm-1 decrease slowly within 2h (< 10%)
Spectra collected every 30 s, at pH 8.0
Relative Time
Absorbance at 1648 cm -1 is stable within 24h
Spectra collected every 30 s, at pH 5.15
Relative Time


Figure 2

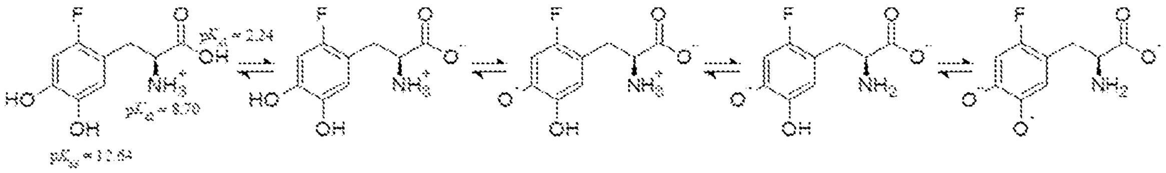

H4L H3L H2L HL L

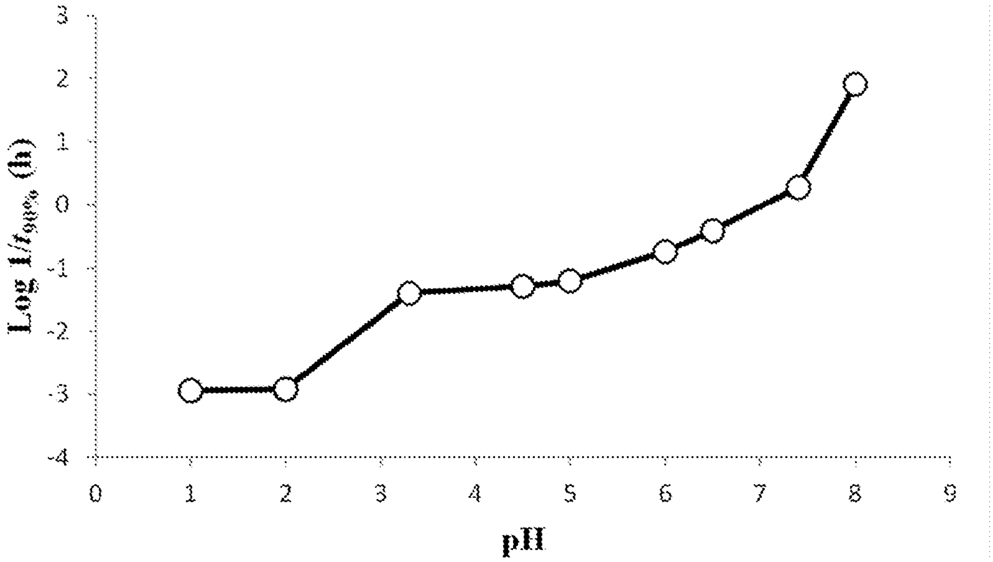


Figure 3

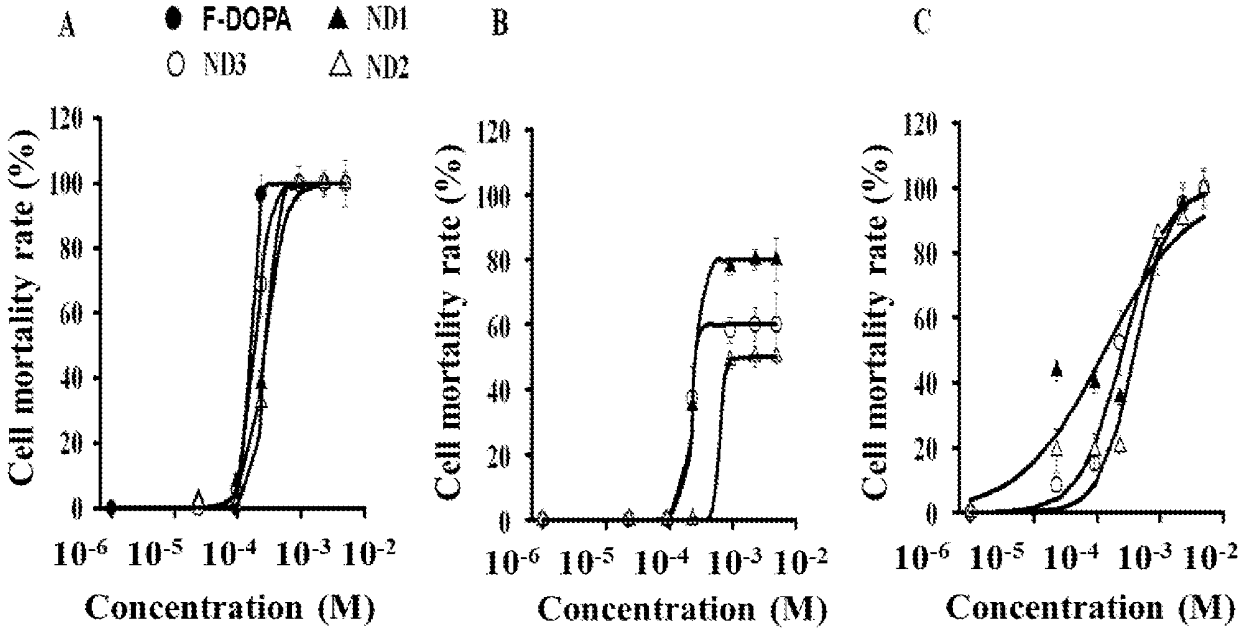


Figure 4

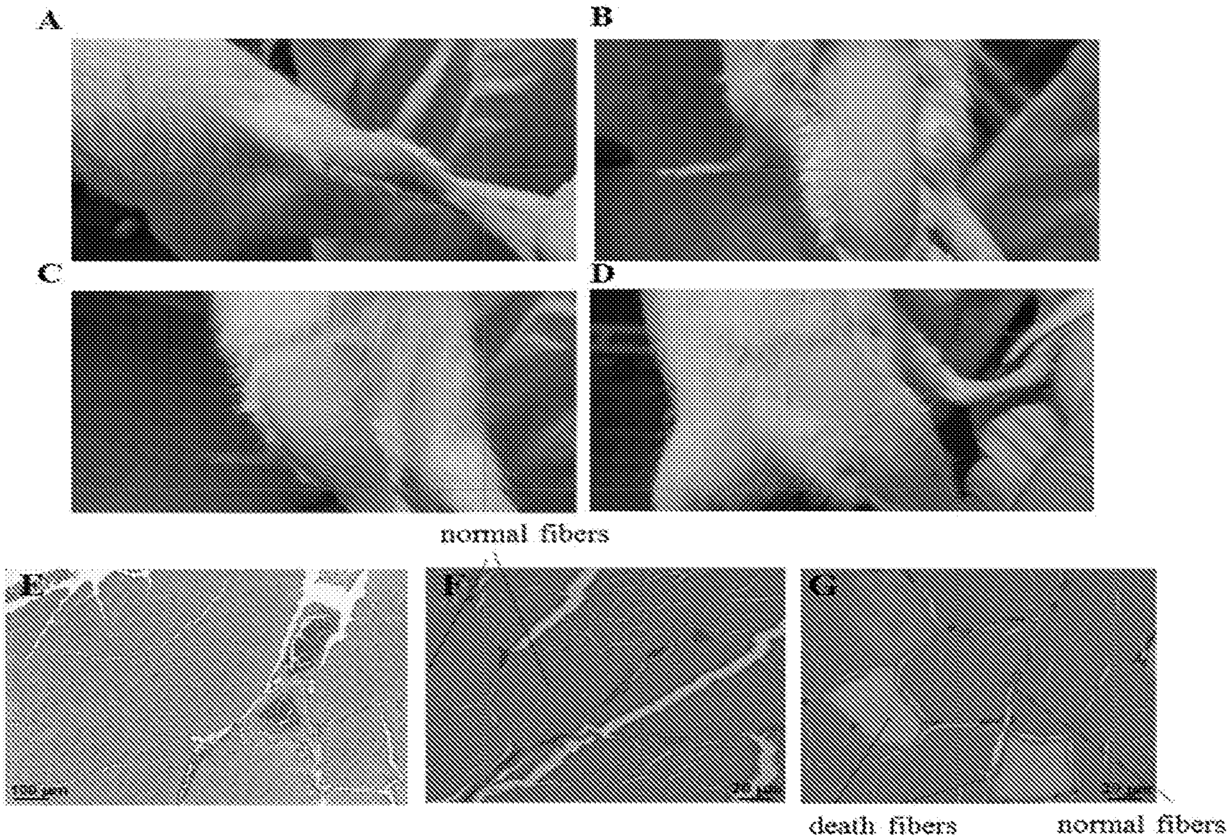


**Figure 5**

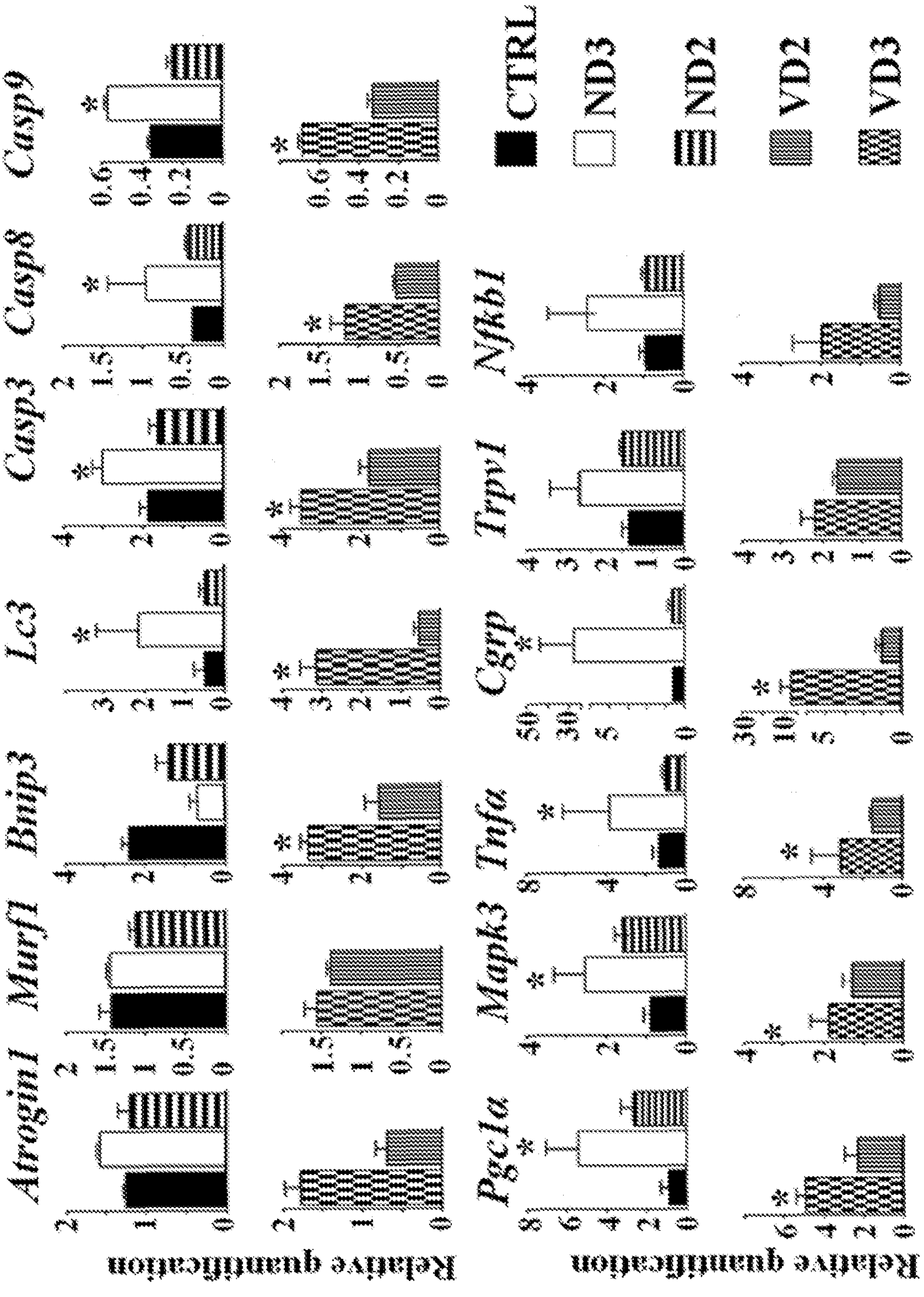
Relative quantification
Relative quantification
Atrogin1
Murf1
Bnip3
Lc3
Casp3
Casp8
Casp9
Pgc1a
Mapk3
Tnfa
Cgrp
Trpv1
Nfkb1
CTRL
ND3
ND2
VD2
VD3

Figure 6

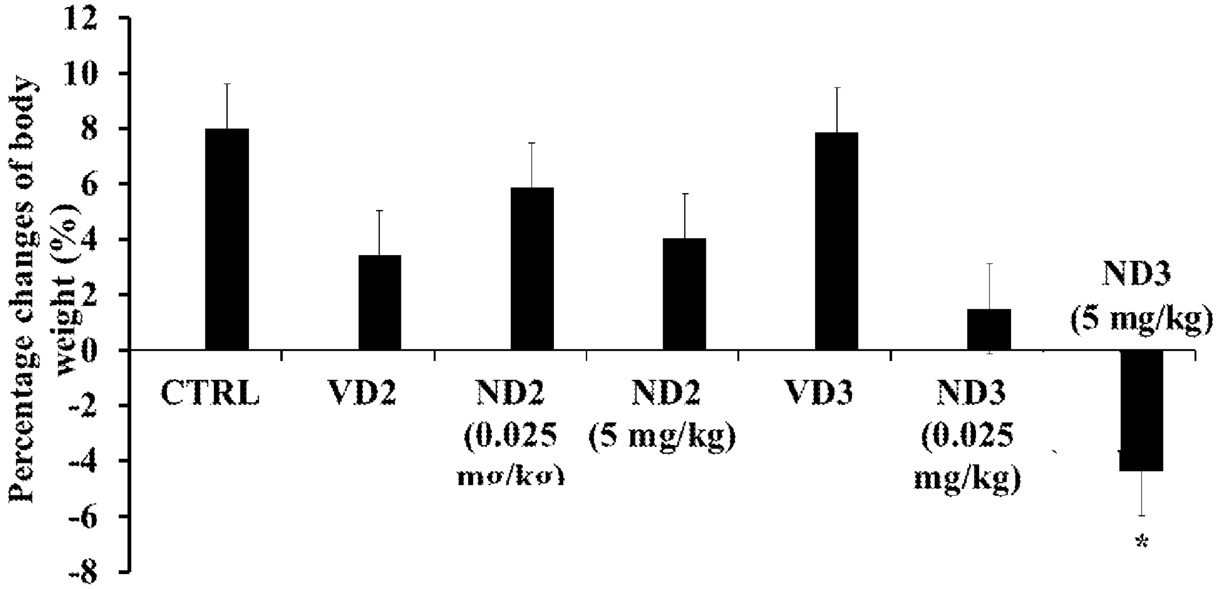


Figure 7

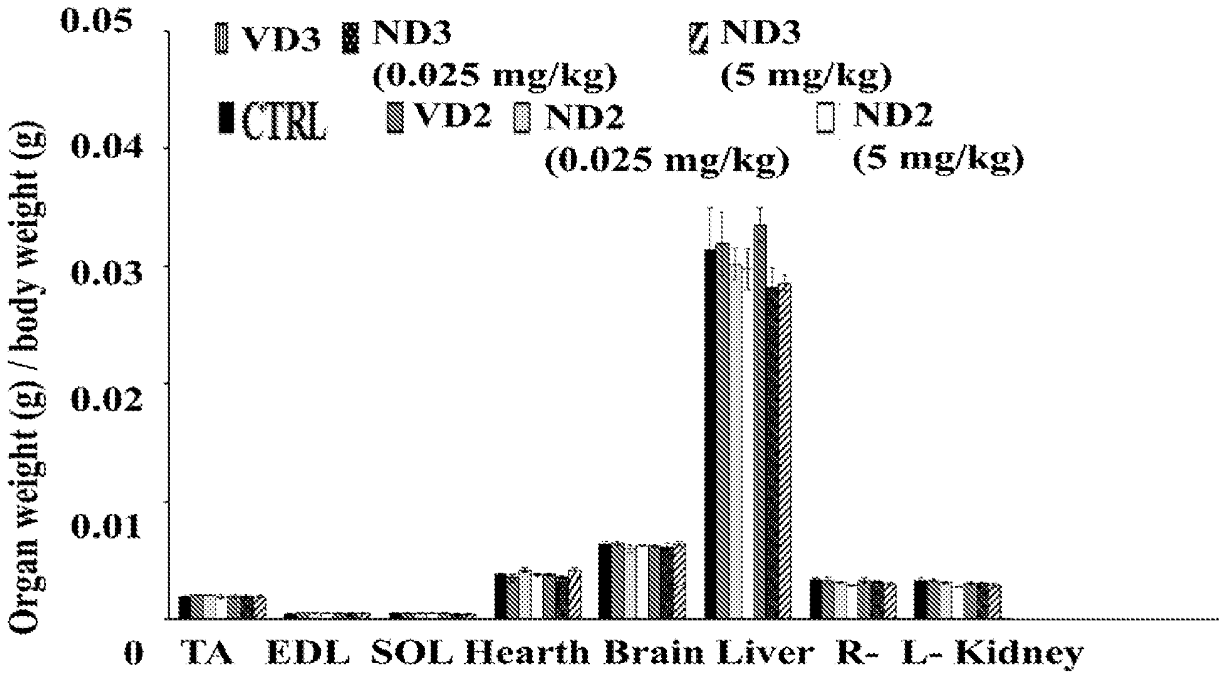


Figure 8

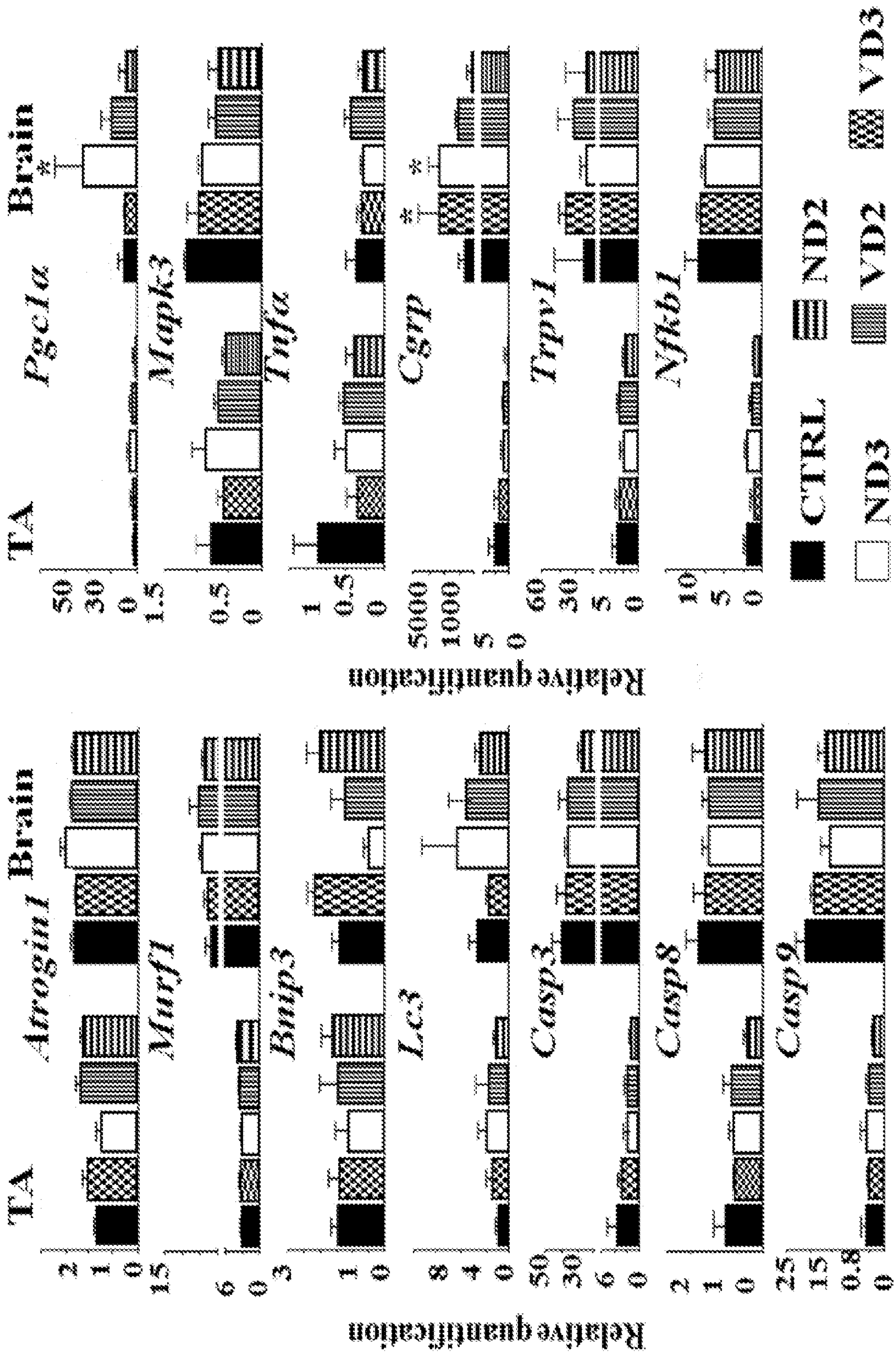
TA
Brain
Atrogin1
Murf1
Bnip3
Lc3
Casp3
Casp8
Casp9
Pgc1a
Mapk3
Tnfa
Cgrp
Trpv1
Nfkb1
Relative quantification
CTRL
ND2
ND3
VD2
VD3

Figure 9

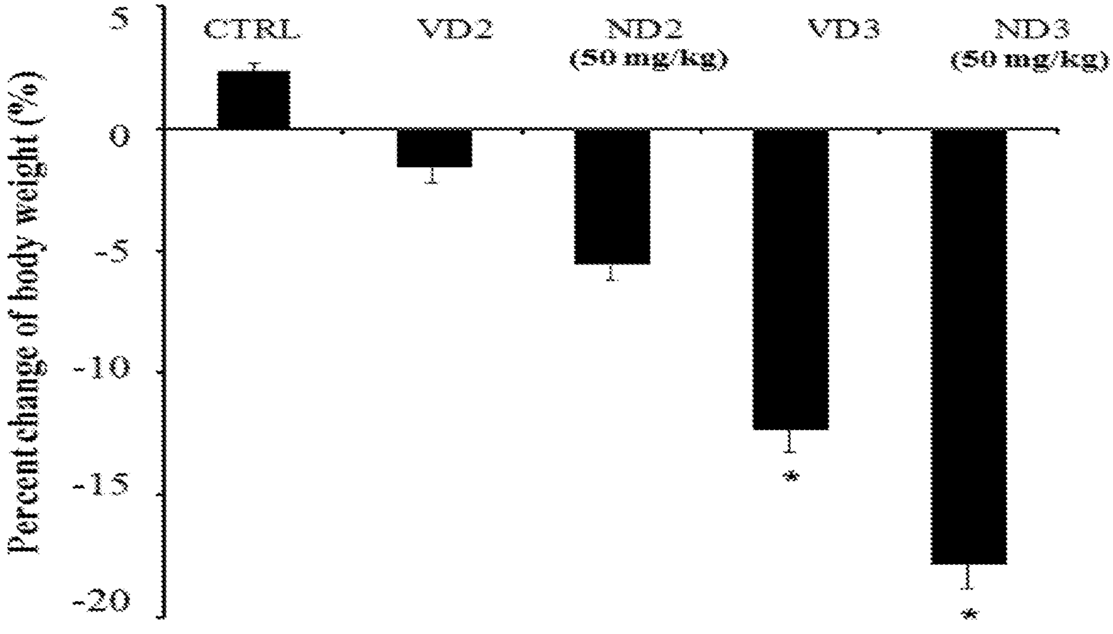


Figure 10

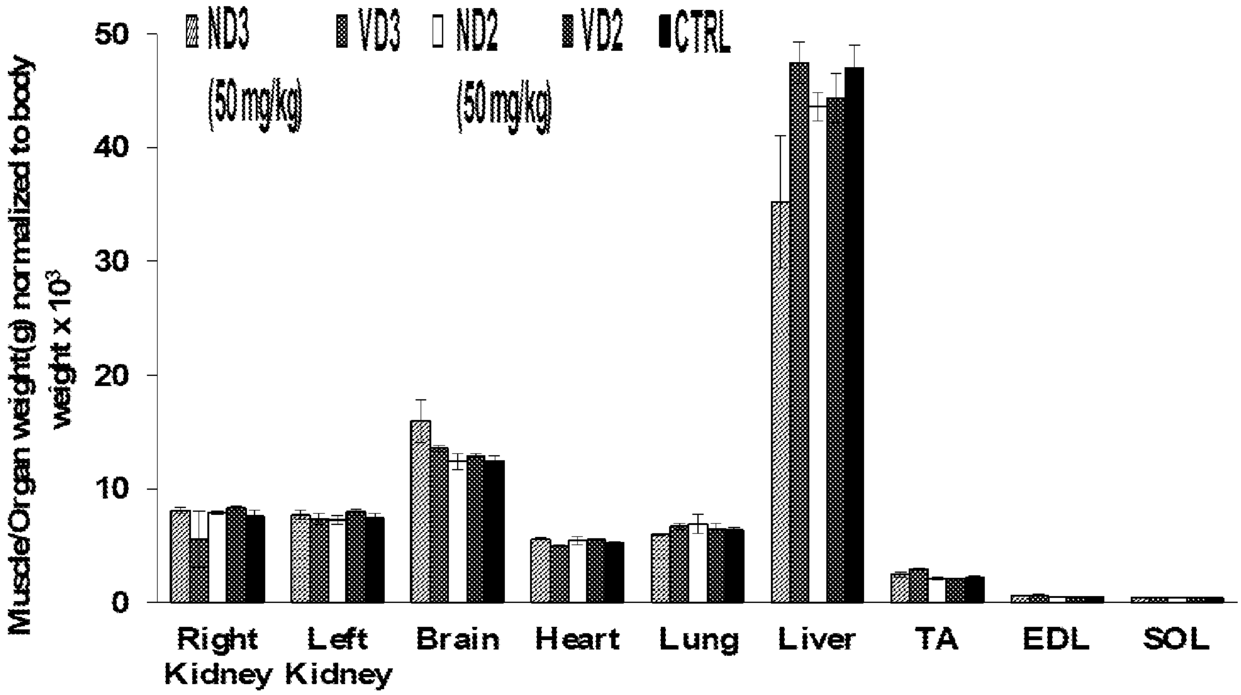


Figure 11

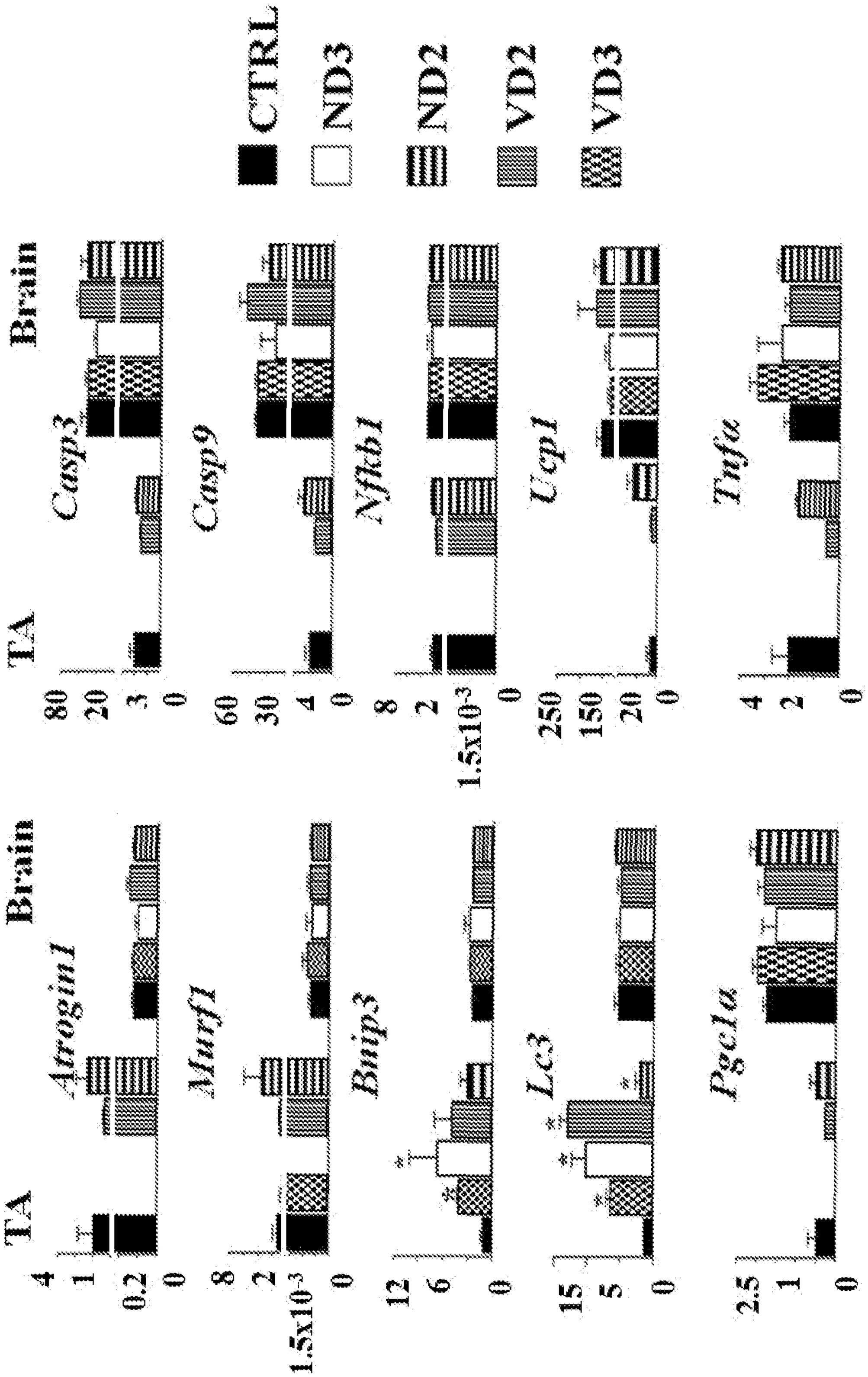

CTRL
ND3
ND2
VD2
VD3
TA
Brain
Atrogin1
Murf1
Bnip3
Lc3
Pgc1a
Casp3
Casp9
Nfkb1
Ucp1
Tnfa


Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2012029085 A